(19) **Europäisches Patentamt / European Patent Office / Office européen des brevets**

(11) **EP 1 001 815 B1**

(12) **FASCICULE DE BREVET EUROPEEN**

(45) Date de publication et mention
de la délivrance du brevet:
**09.11.2011 Bulletin 2011/45**

(21) Numéro de dépôt: **98941540.1**

(22) Date de dépôt: **04.08.1998**

(51) Int Cl.:
*A61K 47/48* (2006.01)          *A61K 39/395* (2006.01)
*C07K 19/00* (2006.01)          *C12N 5/20* (2006.01)

(86) Numéro de dépôt international:
**PCT/FR1998/001740**

(87) Numéro de publication internationale:
**WO 1999/007414 (18.02.1999 Gazette 1999/07)**

(54) **VECTEURS DERIVES D'ANTICORPS POUR LE TRANSFERT DE SUBSTANCES DANS LES CELLULES**

VON ANTIKÖRPERN ABGELEITETE VEKTOREN FÜR DEN TRANSFER VON SUBSTANZEN IN ZELLEN

VECTORS DERIVED FROM ANTIBODIES FOR TRANSFERRING SUBSTANCES INTO CELLS

(84) Etats contractants désignés:
**AT BE CH CY DE DK ES FI FR GB GR IE IT LI LU MC NL PT SE**

(30) Priorité: **04.08.1997 FR 9709972**

(43) Date de publication de la demande:
**24.05.2000 Bulletin 2000/21**

(60) Demande divisionnaire:
**08167774.2**

(73) Titulaires:
  • **INSTITUT PASTEUR**
    **75724 Paris Cedex 15 (FR)**
  • **UNIVERSITÉ PIERRE ET MARIE CURIE (PARIS VI)**
    **75252 Paris Cédex 05 (FR)**

(72) Inventeurs:
  • **TERNYNCK, Thérèse**
    **F-75015 Paris (FR)**
  • **AVRAMEAS, Alexandre**
    **F-94400 Vitry sur Seine (FR)**
  • **BUTTIN, Gérard**
    **F-75013 Paris (FR)**
  • **AVRAMEAS, Stratis**
    **F-75015 Paris (FR)**
  • **SARON, Marie-Françoise**
    **F-75013 Paris (FR)**
  • **BLONDEL, Bruno**
    **F-91440 Bures sur Yvette (FR)**
  • **COUDERC, Thérèse**
    **F-75018 Paris (FR)**
  • **MICHELSON, Susan**
    **F-78590 Noisy (FR)**
  • **ZIPETO, Donato**
    **F-75018 Paris (FR)**

(74) Mandataire: **Desaix, Anne et al**
    **Ernest Gutmann - Yves Plasseraud S.A.S.**
    **3, rue Auber**
    **75009 Paris (FR)**

(56) Documents cités:
**WO-A-97/02840          WO-A-97/32602**
**DE-A- 4 412 629          US-A- 5 296 348**
**US-A- 5 521 291          US-A- 5 606 017**
**US-A- 5 635 383**

  • **ZACK ET AL.: "DNA MIMICS A SELF-PROTEIN THAT MAY BE A TARGET FOR SOME ANTI-DNA ANTIBODIES IN SYSTEMIC LUPUS ERYTHEMATOSUS" JOURNAL OF IMMUNOLOGY, vol. 154, 1995, pages 1987-1994, XP002063314**
  • **ZACK ET AL.: "MECHANISMS OF CELLULAR PENETRATION AND NUCLEAR LOCALIZATION OF AN ANTI-DOUBLE STRAND DNA AUTOANTIBODY" JOURNAL OF IMMUNOLOGY, vol. 157, 1996, pages 2082-2088, XP002063315**
  • **BACCALA ET AL.: "TWO MURINE NATURAL POLYREACTIVE AUTOANTIBODIES ARE ENCODED BY NONMUTATED GERM-LINE GENES" PNAS, vol. 86, 1989, pages 4624-4628, XP002087948**

- SAKANO ET AL.: "TWO TYPES OF SOMATIC RECOMBINATION ARE NECESSARY FOR THE GENERATION OF COMPLETE IMMUNOGLOBULIN HEAVY-CHAIN GENES" NATURE, vol. 286, 1980, pages 676-683, XP002087949
- VLAHAKOS ET AL.: "MURINE MONOCLONAL ANTI-DNA ANTIBODIES PENETRATE CELLS, BIND TO NUCLEI AND INDUCE GLOMEULAR PROLIFERATION AND PROTEINURIA IN VIVO" JOURNAL OF THE AMERICAN SOCIETY OF NEPHROLOGY, vol. 2, 1992, pages 1345-1354, XP002063435
- AVRAMEAS: "POLYREACTIVE ANTI-DNA MONOCLONAL ANTIBODIES AND A DERIVED PEPTIDE AS VECTORS FOR THE INTRACYTOPLASMIC AND INTRANUCLEAR TRANSLOCATION OF MACROMOLECULES" PNAS, vol. 95, mai 1998, pages 5601-5606, XP002087950

**Description**

La présente invention est relative au transfert actif d'haptènes, de protéines, de peptides, d'acides nucléiques et autres molécules dans les cellules. La présente invention concerne plus particulièrement de nouveaux polypeptides capables de pénétrer efficacement dans les cellules, notamment eucaryotes, et d'y véhiculer une substance d'intérêt et susceptibles de constituer de nouvelles compositions antivirales. Cette invention revêt une importance majeure puisqu'elle peut être appliquée à divers domaines, notamment celui de la thérapie génique et des vaccins.

La thérapie génique demeure conditionnée par un nombre considérable de paramètres, parmi lesquels le développement de vecteurs capables de transférer dans le cytoplasme des cellules de l'organisme de l'hôte considéré des principes actifs doués de propriétés spécifiques prédéterminées, en l'absence d'altérations génétiques associées à l'utilisation de ces vecteurs, et sans dégradation de l'activité biologique des principes actifs transférés. On sait qu'à ce jour, en dépit des efforts réalisés pour le développement de vecteurs d'origine virale ou non-virale, toutes ces conditions n'ont pas été remplies de manière satisfaisante.

Par ailleurs, la possibilité de véhiculer efficacement des substances à l'intérieur des cellules est également importante pour toutes les applications des biotechnologies. Ainsi, le transfert de substances dans les cellules in vitro ou ex vivo peut être utilisé soit pour la production de protéines ou de peptides, soit pour la régulation de l'expression de gènes ou encore pour l'analyse des propriétés d'une substance donnée sur ladite cellule. In vivo, le transfert d'une substance dans une cellule peut en outre servir à la création de modèles pour l'étude de pathologies chez les animaux ou également pour étudier l'effet de composés donnés sur un organisme.

La présente invention a donc pour but de mettre à disposition un nouveau type de vecteurs à la fois efficaces et d'une plus grande innocuité que les vecteurs viraux dont l'utilisation est envisagée à ce jour.

Dans la demande de brevet n° WO 97/02840, le demandeur a décrit l'utilisation d'anticorps ou de leurs fragments F(ab')2 et Fab' capables de pénétrer à l'intérieur des cellules vivantes, comme immunovecteurs pour le transfert intracytoplasmique et intranucléaire de substances biologiquement actives. Bien que ces vecteurs présentent une efficacité importante, leur utilisation pourrait, dans certaines applications, présenter une complexité. Ainsi, l'emploi d'anticorps ou de fragments F(ab')2 d'anticorps implique la production de ces molécules à des titres élevés et avec des qualités compatibles avec une utilisation thérapeutique. En outre, l'utilisation de molécules de la taille et de la complexité des anticorps peut représenter un autre inconvénient sur le plan de la mise en oeuvre notamment. Le brevet n° US 5,635,383 illustre un autre type de vecteur complexe, à base de polylysine, pour le transfert d'acides nucléiques dans les cellules.

La présente demande est relative à de nouveaux polypeptides présentant des propriétés avantageuses, à la fois pour le transfert de substances dans les cellules et comme agents antiviraux. Ces polypeptides ont notamment une structure primaire beaucoup plus simple que des anticorps et une taille réduite. En outre, leur préparation est aisée et leurs applications potentielles très variées.

Plus particulièrement, la présente invention résulte de la découverte, par les inventeurs, qu'il est possible d'identifier à partir d'anticorps entiers, des régions limitées porteuses d'une activité de pénétration cellulaire. L'invention découle en outre de la découverte qu'il est possible d'isoler à partir d'anticorps entiers en particulier à partir d'une seule chaîne de ces anticorps, des peptides ou polypeptides doués d'activité de pénétration cellulaire. La présente invention constitue la première démonstration qu'un fragment d'une seule chaîne d'un anticorps peut effectivement pénétrer dans les cellules. La présente invention constitue également la première démonstration qu'un tel fragment est en plus capable, de manière avantageuse, de véhiculer dans ladite cellule une substance d'intérêt, et peut présenter de manière préférentielle une activité antivirale.

La présente invention fournit donc de nouvelles molécules particulièrement adaptées au transfert dans les cellules eucaryotes, particulièrement des cellules de mammifères, de substances biologiquement actives.

Un premier objet de l'invention réside donc plus particulièrement dans un polypeptide caractérisé en ce que :

- il est constitué d'un motif peptidique unique ou répété, et
- il comprend une séquence d'amino-acides lui conférant la capacité de pénétrer dans des cellules et, le cas échéant, d'y véhiculer une substance d'intérêt.

L'invention concerne à cet égard un polypeptide caractérisé en ce que :

- il est constitué d'un motif peptidique unique ou répété,
- il comprend une séquence d'amino-acides constituée d'un ou plusieurs fragment(s) différent(s) d'anticorps, et,
- il est capable de pénétrer dans les cellules.

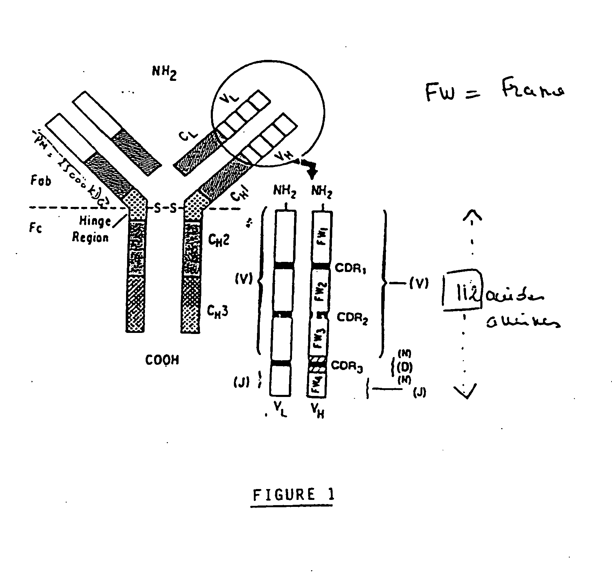

Selon un des modes de réalisation de l'invention, les polypeptides comprennent donc un ou plusieurs fragment (s) différents ou non d'un anticorps. Les anticorps, molécules de la superfamille des immunoglobulines, sont constitués sous leur forme la plus simple de quatre chaînes associées entre-elles (IgG par exemple) : deux chaînes lourdes, dites "Heavy" ou "H", et deux chaînes légères, désignées "Light" ou "L" (Figure 1). Ces quatre chaînes sont donc associées ensemble, post-synthèse, pour former une molécule d'un poids moléculaire de 150.000 Kd environ. La spécificité antigénique des anticorps est assurée par des domaines variables impliquant plusieurs régions d'une chaîne lourde et plusieurs régions d'une chaîne légère (Figure 1).

Les polypeptides peuvent aussi être constitués de séquences provenant des autres représentants des immunoglobulines telles que les IgM.

Chaque chaîne lourde des anticorps est composée de 450 acides aminés environ, et comprend différents domaines, désignés domaine constant (C), domaine variable (V) et domaines de jonction (D et J). A l'intérieur des domaines variables se trouvent des motifs particuliers, désignés CDR ("Complementarity Determining Region") qui peuvent être aisément localisés par alignement de séquences (C. Janeway et P. Travers, 1996, immunobiology, Academic Press, "The Structure of A Typical Antibody Molecule"). On peut aussi se référer pour l'analyse des séquences des régions variables à l'article de T.T. Wu et E. Kabat (J. Exp. Med., 1970, Vol. 132, p. 211-250). Les motifs CDR comportent eux-mêmes des régions hypervariables.

La présente demande résulte de la mise en évidence qu'il est possible d'obtenir. à partir de la structure des anticorps, des régions limitées en taille et de structure simple, possédant des propriétés tout à fait avantageuses. Ainsi, partant d'une molécule complexe (quatre chaînes associées) et de taille importante (150.000 Kd), le demandeur a réussi à construire des polypeptides à chaîne unique, ayant la capacité de pénétrer dans les cellules et d'y véhiculer des substances d'intérêt. Les propriétés des polypeptides de l'invention sont d'autant plus remarquables que leurs séquences correspondent à celles d'un ou plusieurs fragments d'une seule des chaînes d'un anticorps et qu'ils n'ont donc pas besoin, pour être actifs, de régions constantes provenant d'une chaîne lourde et d'une chaîne légère. Les polypeptides selon l'invention obtenus par synthèse chimique ont les mêmes propriétés.

Le terme polypeptide désigne au sens de l'invention une molécule comprenant un enchaînement d'acides aminés, ayant une taille inférieure à 60 acides aminés. Encore plus préférentiellement, il s'agit d'une molécule comprenant un enchaînement de 3 à 60 acides aminés; avantageusement de 3 à 30. Des polypeptides particulièrement préférés comportent avantageusement plus de 10 acides aminés environ. Le polypeptide selon l'invention peut en outre comporter certaines modifications structurales, de nature chimique ou enzymatique par exemple. Ainsi, le polypeptide selon invention peut comporter certains groupes fonctionnels pouvant, par réaction chimique ou enzymatique, réaliser un couplage avec une autre substance. Les polypeptides de l'invention peuvent en outre être modifiés chimiquement afin de les rendre plus résistants à des protéases ou moins visibles au système immunitaire. Les polypeptides selon l'invention peuvent être obtenus par toute méthode connue de l'homme du métier, et notamment par synthèse chimique, par exemple au moyen de synthétiseurs de peptides, ou par fragmentation ou délétion à partir de polypeptides, naturels ou non, de taille supérieure. Ils peuvent en outre être préparés par les techniques de l'ADN recombinant, par expression, dans un hôte cellulaire eucaryote ou procaryote, d'un acide nucléique correspondant. Ils peuvent bien entendu résulter de combinaisons de ces différentes méthodes.

A cet égard, les polypeptides de l'invention peuvent être produits à partir de banques d'acides nucléiques ou de peptides, telles que des banques de synthèse combinatoire.

Le terme "motif peptidique unique" signifie que, contrairement aux anticorps ou aux fragments d'anticorps de type Fab ou F(ab')2 par exemple, les polypeptides de l'invention ne comportent qu'une seule chaîne d'acides aminés. Le terme "motif peptidique répété" désigne le fait que les polypeptides de l'invention peuvent comporter différents blocs peptidiques assemblés entre-eux, éventuellement par voie chimique, pour ne former qu'une seule chaîne.

Le terme "pénétrer" ou "pénétrant" désigne au sens de l'invention un polypeptide capable de passer depuis le milieu extérieur jusque dans le milieu intracellulaire, notamment dans le cytoplasme de la cellule. Cette capacité peut se déterminer de différentes façons, et notamment selon un test de pénétration cellulaire comportant une première incubation du polypeptide étudié en présence de cellules en culture, suivie, après fixation et perméabilisation de ces cellules, d'une révélation de la présence dudit polypeptide à l'intérieur de ladite cellule. La révélation peut être réalisée par une autre incubation avec des anticorps marqués dirigés contre ledit polypeptide et détection, dans le cytoplasme ou à proximité immédiate du noyau ou même au sein de celui-ci, de la réaction immunologique du type antigène-anticorps entre le polypeptide et l'anticorps marqué. La révélation peut également être réalisée en utilisant un polypeptide de l'invention préalablement marqué et en détectant de la présence dudit marquage dans ces compartiments cellulaires. Un tel test de pénétration cellulaire a été décrit par exemple dans la demande n° WO 97/02840.

Comme indiqué ci-avant, la présente invention résulte de la mise en évidence de l'existence de régions réduites d'un anticorps douées de propriétés de pénétration cellulaire et pouvant également jouer le rôle de véhicule pour des substances d'intérêt. Plus particulièrement, les inventeurs ont recherché dans la structure de certains anticorps dits pénétrants tels que ceux décrits dans la demande WO 97/02840, la présence de régions douées de propriétés de pénétration cellulaire et qui pourraient être utilisées comme vecteur à la place des anticorps entiers. Pour cela, les inventeurs ont, dans un premier temps, déterminé la séquence complète des chaînes lourdes et légères de trois anticorps monoclonaux particuliers, désignés J20.8, F4.1 et F14.6. Ces anticorps sont des anticorps anti-ADN, polyréactifs, qui sont produits par les hybridomes déposés à la CNCM sous les numéros I-1605, I-1606 et I-1607 (voir demande de brevet précitée). L'alignement de ces séquences et leur analyse comparative ont fait apparaître les éléments remarquables suivants :

- L'existence d'une région de très forte homologie (65-70 %) dans la région CDR2 de ces trois anticorps, et,
- La présence, dans ces trois anticorps, de régions CDR3 riches en lysine et arginine (acides aminés basiques).

A la vue des ces résultats, et étant donné que la plupart des peptides capables de transport et de localisation nucléaire sont riches en lysine et arginine, les demandeurs ont ensuite synthétisé des séries de polypeptides correspondant à différentes régions de ces anticorps, et notamment aux régions CDR2 et CDR3, ainsi que des constructions hybrides dans lesquelles certaines de ces régions sont fusionnées entre elles (en particulier un polypeptide CDR2-3 portant successivement les régions CDR2 et CDR3). Un résidu biotine a par ailleurs été introduit du côté N-terminal de ces polypeptides, ce qui permet leur détection aisée.

Ces polypeptides ont ensuite été testés pour leur capacité à pénétrer dans les cellules. Les résultats obtenues montrent, de manière tout à fait remarquable, que certains de ces polypeptides possèdent la capacité de pénétrer efficacement dans les cellules. En particulier, les résultats obtenus montrent que l'ensemble des polypeptides qui comprennent tout ou une partie de la région CDR3 sont capables de pénétrer dans les cellules.

Plus préférentiellement, les polypeptides de l'invention sont donc constitués d'une chaîne unique comprenant au moins un fragment de la chaîne lourde d'un anticorps. Ils comprennent au moins un fragment de la région variable de la chaîne lourde d'un anticorps l'invention décrit des polypeptides tels que définis ci-avant comprenant tout ou une partie de la région CDR3 d'un anticorps.

Par ailleurs, les résultats obtenus ont également montré que des polypeptides possédant en outre tout ou une partie de la région CDR2 possédaient également la capacité de pénétrer dans les cellules. A cet égard, des polypeptides dans lesquels sont combinées tout ou partie de la région CDR3 et tout ou partie de la région CDR2 possèdent des capacités de pénétration cellulaire tout à fait remarquables.

Ainsi, selon un autre mode particulier de réalisation, les polypeptides de l'invention comprennent tout ou une partie de la région CDR2 d'un anticorps.

Selon un mode de mise en oeuvre particulièrement intéressant, les polypeptides de l'invention comprennent plus préférentiellement tout ou une partie de la région CDR3 et tout ou une partie de la région CDR2. Ce type de polypeptide est particulièrement avantageux car il est capable de pénétrer massivement à l'intérieur de cellules vivantes.

L'expression "tout ou une partie" telle qu'utilisée dans la présente demande désigne plus particulièrement le fait que les polypeptides de l'invention peuvent comporter soit l'ensemble de la région CDR concernée d'un anticorps, soit une partie seulement de celle-ci, étant entendu que le polypeptide conserve la capacité de pénétrer dans les cellules (homologue fonctionnel). Une partie de région CDR peut notamment consister en une région CDR dépourvue de un ou plusieurs acides aminés terminaux, en particulier de un, deux ou trois acides aminés terminaux. Il peut également s'agir d'une région COR dans laquelle un ou plusieurs résidus internes ont été délétés ou substitués par d'autres acides aminés, de préférence des acides aminés de même nature (acides aminés basiques par exemple). Avantageusement, moins de 15% des résidus internes de la région CDR sont- modifiés.

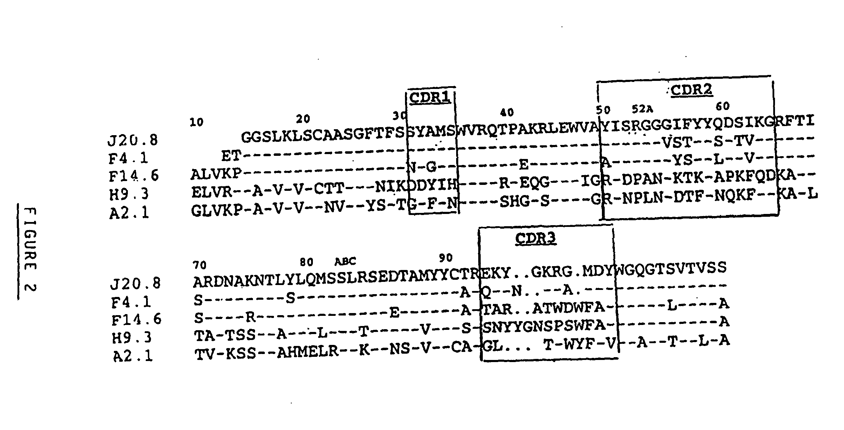

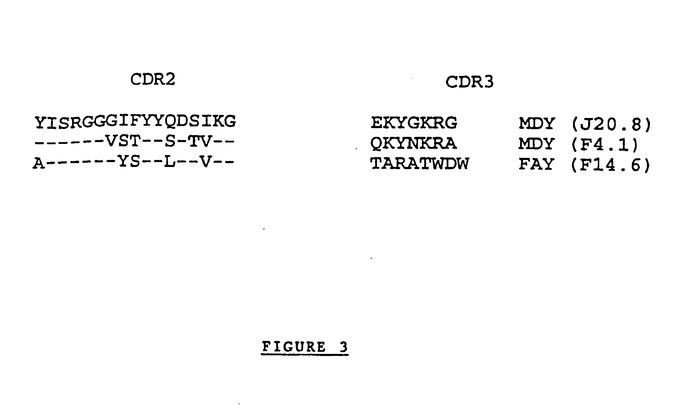

Des polypeptides préférés au sens de l'invention sont donc des polypeptides comportant tout ou une partie d'une région CDR3 d'un anticorps. A titre illustratif, on peut citer par exemple les régions CDR3 de séquence SEQ ID N° 1, 2, 3, 8 ou les séquences représentées sur la figure 2 et sur la figure 3, ou tout homologue fonctionnel.

Les fragments d'anticorps peuvent constituer à eux seuls un polypeptide de l'invention. Ils peuvent également être modifiés, notamment par addition de résidus à une ou plusieurs de leurs extrémités. En particulier, il peut être avantageux le cas échéant d'ajouter des acides aminés qui vont donner au fragment, notamment à la région CDR, une meilleure configuration spatiale. Il peut en outre être avantageux d'ajouter un ou plusieurs acides aminés essentiellement basiques, de type lysine et/ou arginine, afin de stabiliser le polypeptide et d'augmenter son interaction avec les membranes cellulaires. En outre, comme indiqué ci-avant, les polypeptides de l'invention peuvent comporter plusieurs régions d'une chaîne d'anticorps, tel que .par exemple une région CDR2 et une région CDR3. Ces régions peuvent en particulier être fusionnées entre elles ou espacées d'acides aminés tels que décrits ci-avant.

Des polypeptides particuliers au sens de l'invention sont notamment les polypeptides comprenant une région CDR3 d'anticorps ou les polypeptides comprenant essentiellement une fusion entre la région CDR3 d'un anticorps et la région CDR2 d'un anticorps. Des exemples de tels polypeptides sont notamment le polypeptide CDR2-3 dont les séquences sont données dans les exemples.

Les expériences réalisées avec ces polypeptides, notamment les polypeptides comportant la région CDR3, et en particulier ceux comportant la région CDR3 et la région CDR2, montrent de façon claire que :

1) une incubation d'une heure, de cellules PtK2, HeLa ou 3T3 avec le milieu de culture complet (10% de sérum de veau foetal) contenant le polypeptide est suffisante pour que le polypeptide soit transporté massivement dans le cytoplasme de toutes les cellules et dans le noyau d'une grande proportion de ces cellules variable selon la lignée concernée.

2) quand on incube pendant 2 heures les cellules dans le milieu de culture complet contenant des complexes préformés peptide-streptavidine couplée à la peroxydase (PM ≥ 100.000) ou peptide-streptavidine couplée à la phosphatase alcaline (PM ≥ 180.000), on détecte les enzymes correspondants au niveau du cytoplasme de toutes les cellules de la culture et faiblement jusqu'à intensément dans la majorité des noyaux de ces cellules. Aucune coloration intracellulaire n'est observée quand les cellules sont incubées en présence de streptavidine couplée à là peroxydase, streptavidine couplée à la phosphatase alcaline ou avec la streptavidine ou les enzymes sous leurs formes natives.

Les polypeptides de l'invention notamment de type CDR2-3 ainsi que leurs complexes peptide-streptavidine-enzyme sont transportés en grandes quantités dans une large proportion des cellules périphériques humaines et particulièrement dans les lymphocytes T activés.

D'une manière générale, les polypeptides selon l'invention peuvent être construits selon différentes techniques connues de l'homme du métier (supra), en partant de tout anticorps donné, notamment de tout anticorps monoclonal donné.

Préférentiellement, les polypeptides de l'invention sont obtenus par synthèse chimique ou sont construits à partir d'un fragment ou plusieurs fragments d'un ou plusieurs anticorps pénétrant(s), de préférence d'un anticorps monoclonal pénétrant. L'existence d'anticorps capables de pénétrer à l'intérieur des cellules et en particulier de noyaux de lymphocytes humains lorsque ces cellules sont incubées in vitro dans un milieu de culture contenant un sérum provenant de malades atteints de lupus érythémateux disséminé (LED), a été rapportée pour la première fois par Alarcon-Segovia et al. en 1978 (Nature, 271). Récemment, ce type d'anticorps a été détecté chez la souris lupique MRL lpr/lpr, mais également chez la souris NZB présentant un syndrome de maladie hémolytique autoimmune et même chez la souris normale BALB/c. Certains anticorps monoclonaux, préparés à partir de la rate de ces souris, se sont avérés capables de pénétrer in vitro dans le noyau de cellules maintenues en culture (Vlahakos et al., J. Am. Soc. Nephrol. 2 (1992) 1345; Eyal Raz et al., Eur. J. Immunol. 23 (1993) 383). De plus, il a été montré que ces anticorps sont aussi capables, lorsqu'ils sont injectés chez la souris, de pénétrer dans plusieurs types de cellules, pour se retrouver dans leurs noyaux (Okudaira et al., Arthritis and Rheumatism, 30 (1987) 669).

De manière générale, tout anticorps peut être sélectionné en vue de déterminer son caractère pénétrant. En particulier, cette sélection peut être réalisée par exemple par un test de pénétration cellulaire comportant une première incubation de l'anticorps étudié en présence de cellules, notamment de cellules dans lesquelles il est envisagé de véhiculer une substance d'intérêt, suivie après fixation et perméabilisation de ces cellules, de la révélation de la présence ou non de cet anticorps dans la membrane plasmique, le cytoplasme ou à proximité immédiate du noyau ou même au sein de celui-ci. La révélation peut être réalisée par exemple par incubation avec un deuxième anticorps marqué, dirigé contre l'anticorps testé, suivie de la détection de la réaction immunologique du type antigène-anticorps entre ces deux anticorps. Un tel test a été décrit en détail par exemple dans la demande de brevet n° FR N°9508316.

Encore plus préférentiellement, le fragment d'anticorps utilisé pour la_ construction d'un polypeptide de l'invention est un fragment d'un anticorps polyréactif, notamment pénétrant et polyréactif. Un anticorps polyréactif est un anticorps capable de reconnaître plusieurs antigènes différents. Généralement, de tels anticorps ont une affinité particulièrement élevée pour un type d'antigène particulier et sont capables de reconnaître, avec une affinité moindre, un ou plusieurs autres antigènes. La polyréactivité des anticorps peut être mise en évidence par toute méthode immunologique classique, telle que par exemple la méthodologie décrite par Sibille et al. (Eur. J. Immunol. 1997, 27:1221-1228).

De manière avantageuse, les anticorps polyréactifs utilisés dans la construction des polypeptides de l'invention sont capables de réagir avec des acides nucléiques, libres ou complexés avec des protéines (anticorps anti-ADN). Cette propriété peut être mise en évidence par la technique ELISA ou par passage des anticorps sur une colonne ou tout autre support sur lequel de l'ADN a été préalablement immobilisé. Les anticorps anti-ADN sont ainsi retenus sur le support et peuvent être élués et isolés selon les techniques classiques. Généralement, les anticorps anti-ADN utilisés dans le cadre de l'invention présentent une avidité pour l'ADN de l'ordre de $1 \times 10^6 M$ à $2 \times 10^7 M$. Préférentiellement, ces anticorps reconnaissent l'ADN génomique: X Dans un mode particulier, les anticorps utilisés sont des anticorps polyréactifs qui reconnaissent l'ADN génomique de cellules hématopoïétiques humaines. Encore plus préférentiellement, il s'agit d'anticorps capables de réagir avec des acides nucléiques et reconnaissant entre autres des protéines telles que Tat du rétrovirus VIH, et/ou des constituants de surface cellulaire et du cytosquelette des cellules.

Pour la construction d'un polypeptide de l'invention, l'anticorps ainsi retenu est ensuite utilisé comme suit :

a) si la séquence de la région variable de la chaîne lourde ou légère de cet anticorps n'est pas accessible, celle-ci est dans un premier temps déterminée. Pour cela, les techniques classiques de séquençage peuvent être mises en oeuvre, comme illustré dans les exemples.

b) les régions CDRs sont localisées par alignement de la séquence avec d'autres séquences de chaînes d'anticorps, ou par toute autre technique,

c) des fragments de cette séquence sont préparés, ou, des polypeptides correspondants à des régions de cette séquence sont synthétisés et, le cas échéant, assemblés. Pour cela, toute technique classique connue de l'homme du métier peut être utilisée (séquenceurs de peptides, utilisation d'enzyme de restriction, de ligases, etc).

d) le fragment ainsi obtenu est éventuellement modifié par addition, délétion ou substitution d'acides aminés,

e) la capacité du polypeptide ainsi obtenu de pénétrer dans les cellules est ensuite testée dans les conditions décrites plus haut, ainsi que la capacité de véhiculer des substances telles que la fluorescéine ou la peroxydase.

Eventuellement, les étapes c), d) et e) ou d) et e) sont répétées de manière à améliorer l'efficacité de pénétration ou les propriétés générales des polypeptides de l'invention.

Dans une étape supplémentaire ultérieure f), le polypeptide ainsi obtenu, ayant la capacité de pénétrer dans les cellules, est ensuite utilisé dans une réaction de couplage avec une substance donnée pour générer un vecteur tel que défini ci-après.

Une alternative à cette méthode réside dans l'utilisation d'une ou plusieurs banques d'acides nucléiques ou de peptides comme matériel de départ. Ainsi, plutôt que de partir de la séquence d'un anticorps, il est possible de construire, par exemple par chimie combinatoire, des banques de peptides ou d'acides nucléiques codant pour des peptides représentant des homologues fonctionnels de régions CDR2 ou CDR3 d'anticorps.

Ensuite, les peptides ou combinaisons de peptides ou d'acides nucléiques de ces banques sont préparés, éventuellement modifiés et testés pour leur activité, selon les étapes c) à e) du procédé décrit ci-avant.

Préférentiellement, dans l'étape c), du procédé décrit ci-dessus, les fragments préparés comportent tout ou une partie de la région CDR3 d'un anticorps.

Dans l'étape d), les modifications peuvent consister par exemple à introduire certains acides aminés supplémentaires, soit simplement pour des raisons techniques (facilité de synthèse, couplage entre différentes régions, etc) soit pour des raisons structurales ou physicochimiques. S'agissant d'acides aminés de "remplissage", on utilise avantageusement des acides aminés relativement neutres sur le plan structural et physicochimique. S'agissant de raisons structurales, comme indiqué ci-avant, l'addition de résidus peut améliorer la conformation du polypeptide et ainsi, potentialiser son activité. Par exemple, en introduisant des séquences de facteurs de localisation nucléaire (FLN), on peut augmenter leurs potentialités de transfert intranucléaire. Par ailleurs, il peut également être souhaitable d'augmenter le caractère basique des polypeptides.

A cet égard, pour améliorer les propriétés de compaction des polypeptides de l'invention, notamment vis-à-vis des acides nucléiques, des polypeptides ont été construits portant du côté N-terminal des résidus lysine. Avantageusement, le nombre de résidus lysine est inférieur à 30, encore plus préférentiellement, entre 10 et 20.

Les résultats présentés dans les exemples confirment les propriétés de pénétration de ces polypeptides, et leur capacité à véhiculer efficacement des substances d'intérêt, notamment des acides nucléiques.

Quels que soient les ajouts effectués, le polypeptide selon l'invention tel que préparé par exemple selon la stratégie décrite ci-dessus comporte préférentiellement au plus 100 acides aminés. Encore plus préférentiellement, il comprend de 3 à 60 acides aminés et, de préférence, de 3 à 40 acides aminés.

Un autre objet de l'invention concerne l'utilisation d'un polypeptide tel que défini ci-dessus pour le transfert de substances dans les cellules, in vitro, ex vivo ou in vivo.

Un autre objet de l'invention concerne un vecteur pour le transfert d'une substance dans une cellule caractérisé en ce qu'il comprend un polypeptide tel que défini ci-avant auquel est couplée ladite substance.

Selon un mode de réalisation de l'invention, le polypeptide comprend une séquence d'acides aminés lui conférant la capacité de pénétrer dans des cellules et lui permettant d'y véhiculer des substances d'intérêt biologique qui lui sont associées, par exemple des haptènes ou des macro-molécules comportant des centaines à des milliers de kD, tels que des substances médicamenteuses, des protéines ou des acides nucléiques.

La séquence d'acides aminés et les substances d'intérêt thérapeutique associées peuvent par exemple être couplées ou liées par l'intermédiaire de liaisons covalentes ou non-covalentes.

Un polypeptide selon l'invention est avantageusement constitué de peptides ou de macromolécules ayant la capacité à pénétrer à l'intérieur des cellules vivantes, et plus particulièrement à partir de dérivés peptidiques d'anticorps ou de fragments d'anticorps tels que décrits dans la demande de brevet WO 97/02840 ou à partir d'autres peptides comprenant une ou des parties hypervariables d'anticorps, ou encore de molécules synthétiques, non apparentées directement à une structure de type anticorps, susceptibles d'être obtenues par exemple par criblage d'une banque peptidique sur le critère de pénétration dans les cellules.

Un polypeptide particulier selon l'invention est donc composé d'un motif peptidique unique ou répété, et comprend une séquence d'acides aminés lui conférant la capacité de pénétrer dans des cellules et d'y véhiculer une substance d'intérêt, cette séquence étant susceptible d'être obtenue par criblage d'une banque peptidique sur le critère de pénétration dans les cellules. Les conditions de criblage de telles banques ont été décrites ci-avant.

Un autre polypeptide particulier selon l'invention est composé d'un motif peptidique unique ou répété, et comprend une séquence d'acides aminés lui conférant la capacité de pénétrer dans des cellules et d'y véhiculer une substance d'intérêt, cette séquence étant composée d'un peptide comprenant une ou des parties hypervariables d'anticorps.

La substance couplée peut être tout produit d'intérêt, notamment biologique, pharmaceutique ou agro-alimentaire. Il peut s'agir en particulier d'un acide nucléique, tel que par exemple un acide ribonucléique ou un acide désoxyribonucléique. Cet acide nucléique peut en outre être d'origines diverses, et notamment humaine, virale, animale, eucaryote ou procaryote, de plante, synthétique, etc. Cet acide nucléique peut par ailleurs avoir une taille variable, allant du simple oligonucléotide au génome ou fragment de génome. Il peut s'agir notamment d'un génome viral ou d'un plasmide. La substance peut également être une protéine, telle que par exemple une enzyme, hormone, cytokine, apolipoprotéine, facteur de croissance, etc. Un type particulier de substance est représenté par les antigènes. Comme indiqué ci-après, les polypeptides de l'invention permettent en effet, de manière avantageuse, de jouer le rôle d'adjuvant et de stimuler la réponse immunitaire dirigée contre un antigène.

De manière plus générale, la substance peut être tout principe actif de médicament, qu'il s'agisse d'un produit chimique, biochimique ou synthétique.

Pour permettre son transfert dans une cellule, ladite substance est donc couplée à un polypeptide de l'invention.

Le terme "couplé" signifie au sens de l'invention tout type d'interaction permettant une association physique entre la substance et le polypeptide. Il est préférable cependant que l'interaction soit suffisamment stable pour que le vecteur ne se dissocie pas avant la pénétration cellulaire. Pour cette raison, un couplage préféré au sens de l'invention est un couplage covalent.

Le couplage covalent peut être réalisé par différentes techniques connues de l'homme du métier. En particulier, il peut s'effectuer par des liaisons de type maléimide, succinimide, peptidique, disulfide et thioether. On peut se référer à l'ouvrage "Bioconjugate Techniques de Greg. T._ HERMANSON (Academic Press, 1996].

Une méthode particulière consiste par exemple à ajouter, à une extrémité d'un polypeptide de l'invention, un résidu cystéine qui peut être aisément utilisé pour des liaisons disulfure, thioether, amine ou acide. Une autre approche consiste à coupler chimiquement un groupement biotinyl, qui permet de coupler ensuite toute substance liée à la streptavidine. Le couplage peut encore être effectué par l'intermédiaire de la p-benzoquinone (FR N° 7537392 et brevet américain N° 4 925 921 par exemple).

De manière générale, tout procédé de couplage chimique, biochimique, enzymatique ou génétique connu dans la littérature peut être employé.

Par ailleurs, un vecteur selon l'invention peut comporter un polypeptide tel que décrit ci-dessus auquel sont couplées plusieurs substances, identiques ou différentes.

Les exemples présentés ci-après établissent de façon claire que les polypeptides de l'invention possèdent la capacité non seulement de pénétrer dans les cellules, mais également d'y véhiculer des substances d'intérêt. Les exemples montrent notamment le transport de protéines, de type enzyme. Il est entendu que les enzymes peuvent être substituées par toute autre molécule d'intérêt telle que acides nucléiques, peptides, substances médicamenteuses, dans les mêmes conditions.

A cet égard, les exemples montrent également la capacité des peptides de l'invention à transférer des acides nucléiques dans des cellules. Pour cette application particulière, le couplage entre le peptide et l'acide nucléique est généralement un couplage non-covalent, basé sur des interactions ioniques, électrostatiques ou de type Van der Waals. Plus particulièrement, pour une utilisation pour le transfert d'acides nucléiques dans les cellules, un peptide de l'invention comporte avantageusement une région constituée d'acides aminés basiques, de type lysine par exemple, permettant de former un complexe (polyplexe) avec les acides nucléiques chargés négativement. Ainsi, un mode particulier de mise en oeuvre de l'invention réside dans l'utilisation d'un peptide tel que défini ci-avant, portant une région polylysine, pour le transfert d'acides nucléiques (i.e., de plasmides, cosmides, fragments linéaires, gènes, antigènes, antisens, oligonucléotides, etc.) dans les cellules. Un objet particulier de l'invention comprend donc un peptide comprenant une région polylysine et une région dérivée d'un anticorps polyréactif pénétrant, et capable de pénétrer dans les cellules. Plus particulièrement, la région polylysine comprend avantageusement de 5 à 30 résidus lysine, préférentiellement de 5 à 20, qui sont avantageusement non interrompus par d'autres résidus. Concernant la région dérivée de l'anticorps pénétrant, elle peut être définie comme ci-avant. Un tel polypeptide comprend avantageusement moins de 100 résidus, comme expliqué ci-avant.

D'autre part, le fait que les polypeptides selon l'invention permettent le transport massif des protéines à l'intérieur des cellules, a par ailleurs incité le demandeur à examiner la possibilité de les utiliser comme agent de transport intracellulaire d'antigènes, leur conférant un effet adjuvant et conduisant à augmenter la réponse immunitaire contre ces antigènes. Ainsi, des souris ont reçu plusieurs injections avec le conjugué streptavidine-peroxydase seul ou complexé avec un polypeptide de l'invention. Les résultats obtenus montrent que l'emploi d'un polypeptide de l'invention permet d'augmenter, en moyenne de 4 à 8 fois le titre des anticorps anti-streptavidine et anti-peroxydase.

L'invention est également relative à un procédé pour transférer une substance dans une cellule in vitro, ex vivo ou in vivo comprenant :

- le couplage entre ladite substance et un polypeptide tel que défini ci-avant, et,
- la mise en contact de la cellule avec le produit dudit couplage.

Pour une utilisation in vitro ou ex vivo, la mise en contact peut être réalisée par simple incubation des cellules avec le produit du couplage (vecteur). Pour une utilisation in vivo, la mise en contact est généralement réalisée par administration du produit du couplage (vecteur) dans l'organisme considéré. Comme indiqué ci-avant, lorsque le couplage est de type covalent, le vecteur peut comporter une ou plusieurs molécules d'intérêt. Par ailleurs, pour un couplage de type non-covalent, par exemple pour les acides nucléiques, le vecteur est généralement formé par incubation du peptide et des acides nucléiques dans un milieu permettant leur complexation. Les quantités respectives des partenaires sont aisément adaptables par l'homme du métier, en fonction de la nature du peptide (longueur et charge), de l'acide nucléique, et du type cellulaire. A titre d'exemple le couplage peut être réalisé à des concentrations en peptide variant de 0,01 à 100 nmoles de peptide par μg d'acide nucléique, de préférence de 0,01 à 10 nmoles/μg. Par ailleurs, dans le procédé de l'invention, il peut être avantageux d'utiliser en outre un agent stabilisant ou facilitateur, tel le glycérol. Ainsi, les résultats présentés dans les exemples montrent que, en présence de glycérol, l'efficacité de transfection des acides nucléiques peut être améliorée d'un facteur proche de 40. Ainsi, un mode particulier de réalisation du procédé de l'invention comprend la mise en contact des cellules avec le produit du couplage en présence d'un agent stabilisant, notamment de glycérol. Avantageusement, la transfection est réalisée in vitro ou ex vivo en présence de glycérol, à des concentrations variant de 0,1 à 2 M par exemple. Il est entendu que ces concentrations peuvent être adaptées par l'homme du métier.

Un autre objet de l'invention réside par ailleurs dans une cellule, notamment eucaryote, contenant un polypeptide ou un vecteur tel que défini ci-avant. Cette cellule est avantageusement une cellule de mammifère, notamment une cellule animale ou humaine. Il peut s'agir en particulier d'une cellule du système hématopoïétique, telle qu'une cellule progéniteur ou cellule souche ou une cellule lymphocytaire (T, B). Il peut aussi s'agir de cellules présentatrices de l'antigène telles que par exemple les macrophages ou les cellules dendritiques.

D'autre part, les inventeurs ont également montré que les polypeptides de l'invention et les anticorps polyréactifs sont doués de propriétés biologiques propres. L'invention montre notamment que ces polypeptides (dérivés peptidiques) ou anticorps, sont capables d'induire un effet biologique distinct de leur capacité de vectorisation de substances actives. De manière inattendue, la présente invention montre en particulier que ces anticorps et dérivés peptidiques sont capables, par eux-mêmes, d'exercer une activité antivirale sur différentes populations cellulaires et sur différents types de virus.

La présente invention concerne donc également une nouvelle approche pour inhiber l'infection et/ou la réplication virale dans les cellules. La présente invention concerne en particulier l'utilisation d'anticorps ou fragments d'anticorps particuliers à titre d'agents antiviraux, notamment pour l'inhibition de l'infection et/ou de la réplication virale dans les cellules. L'invention décrit également une nouvelle méthode de traitement de cellules pour les rendre plus résistantes à l'infection et/ou à la réplication virale. L'invention concerne également des populations de cellules traitées par des anticorps ou polypeptides, moins sensibles à l'infection et/ou la réplication virale. Cette propriété peut être mise en oeuvre in vitro, ex vivo ou in vivo, éventuellement en combinaison avec d'autres agents, pour diminuer l'infection et le développement de virus, notamment l'infection et/ou la réplication du virus de l'immunodéficience acquise humaine, du poliovirus, du virus de l'herpès ou du cytomégalovirus par exemple.

Un autre objet de invention concerne donc l'utilisation, pour la préparation d'une composition antivirale, d'un ou plusieurs polypeptides tels que définis ci-avant. L'invention concerne plus particulièrement l'utilisation, pour la préparation d'une composition antivirale, d'un ou plusieurs anticorps ou fragments d'anticorps, caractérisée en ce que lesdits anticorps ou fragments d'anticorps sont polyréactifs et sont susceptibles de se lier à l'acide nucléique et préférentiellement à l'ADN.

Plus particulièrement, une composition antivirale au sens de l'invention consiste en une composition capable d'inhiber l'infection d'une cellule cible par un virus et/ou la réplication d'un virus dans une cellule cible.

Comme indiqué ci-avant, cet aspect de la présente invention découle de la mise en évidence des propriétés biologiques inattendues des polypeptides décrits ci-avant et, plus généralement, de certains anticorps polyréactifs, c'est-à-dire susceptibles de reconnaître plusieurs antigènes, est présentant plus spécifiquement la capacité de lier l'ADN.

Un objet plus particulier de l'invention, concerne donc l'utilisation de fragments d'anticorps revendiqués ou polypeptides dérivants anti-ADN et pénétrants, comme agent antiviral.

Comme indiqué ci-avant, les fragments d'anticorps, polyréactifs utilisés sont préférentiellement capables de reconnaître au moins un antigène protéique d'origine cellulaire et/ou virale. Plus particulièrement, ces fragments d'anticorps reconnaissent, en plus de l'ADN, au moins un antigène viral tel qu'un antigène de protéine d'enveloppe virale. Un mode de réalisation particulier de l'invention comprend l'utilisation de ces fragments d'anticorps capables de lier une protéine ou un peptide du virus de l'immunodéficience acquise humaine (VIH). Cette propriété peut être testée par toute technique d'immunologie classique. Ainsi, il est possible d'immobiliser sur tout support approprié (plaque, colonne, bille, etc.) des protéines ou peptides du VIH, et d'incuber ce support avec les anticorps. La formation d'un complexe antigène-anticorps peut ensuite être détectée par toute technique classique (immunofluorescence, réaction enzymatique, etc.).

Généralement, les anticorps anti-ADN. utilisés dans le cadre de l'invention présentent une avidité pour des protéines ou peptides du VIH de l'ordre de $10^6$ à $10^7$M.

Préférentiellement, les anticorps utilisés présentent la capacité de lier les protéines Tat et/ou rev du VIH, plus préférentiellement encore la protéine Tat ou un peptide de cette protéine. A titre de peptide particulier on peut citer les peptides comprenant les résidus 22-37 et 46-60 de la protéine Tat du VIH.

Dans cette application antivirale, les anticorps utilisés peuvent être des anticorps polyclonaux ou monoclonaux. Des anticorps polyclonaux peuvent être isolés directement à partir du sérum de sujets (immunisés ou non, sains ou pathologiques), par prélèvement de sang et isolement des anticorps en présence d'immunoadsorbants (par exemple protéine A couplée sur un support de type sépharose). Des anticorps polyclonaux utilisables dans l'invention peuvent notamment être obtenus à, partir de sérum d'animaux sains ou pathologiques, en particulier de rongeurs, notamment de souris. Il peut s'agir plus particulièrement de souris lupiques auto-immunes, qui présentent des taux élevés d'anticorps naturels reconnaissant divers antigènes. Les anticorps polyclonaux peuvent également être obtenus à partir de sérum humains, par exemple à partir de patients atteints de lupus érythémateux disséminé, qui sont également connus pour présenter un taux d'anticorps polyréactifs naturels élevés. S'agissant d'anticorps monoclonaux, ils peuvent être préparés selon les techniques classiques d'immunologie, par prélèvement des splénocytes d'animaux immunisés ou non, sains ou pathologiques, fusion avec des cellules de myélome, puis dilution clonale et sélection des hybridomes producteurs des anticorps. Ces techniques sont bien documentées dans la littérature, comme notamment dans S.L. Morriso and V.T. Oi, in Advances in Immunology (1989) 44: 65-92 ; J.G.R. Hurrell, Monoclonal Hybridoma Antibiotics : techniques and Applications, CRC Press 1982. Ces techniques sont également illustrées dans les exemples. Les anticorps polyréactifs monoclonaux peuvent également être synthétisés artficiellement ou humanisés à partir de monoclonaux animaux.

Les anticorps utilisés peuvent être des immunoglobulines de différents types, et en particulier des IgG ou des. IgM. Il est entendu que d'autres types d'anticorps peuvent également être mis en oeuvre (IgE, IgA, etc.) dès lors qu'ils présentent les propriétés requises.

D'autre part, comme indiqué ci-avant, il est également possible.

Comme indiqué ci-avant, cet aspect de la présente invention résulte en partie de la mise en évidence des propriétés antivirales de tels polypeptides, anticorps et fragments, et notamment de leur capacité à inhiber la réplication virale dans une cellule cible ou l'infection d'une cellule cible par un virus. Le terme "infection" désigne la pénétration du virus ou du génome viral dans la cellule cible, et le terme "réplication" désigne essentiellement la réplication du génome viral dans ladite cellule cible.

La présente invention peut être mise en oeuvre pour inhiber le cycle de différents virus, et plus particulièrement de virus à ARN (rétrovirus) ou de virus à ADN. En outre, il peut s'agir de virus ayant un tropisme pour l'homme ou pour différents animaux, en particulier mammifères (chiens, chats, lapins, bovins, etc.). Plus préférentiellement, la présente invention permet l'inhibition de virus tels que le virus de l'immunodéficience acquise humaine (VIH), le poliovirus, le virus de l'herpès ou le cytomégalovirus (CMV).

Un mode particulier de mise en oeuvre de l'invention comprend l'utilisation, pour la préparation d'une composition destinée à inhiber l'infection d'une cellule cible par le (VIH) et/ou la réplication du VIH dans une cellule cible, d'un ou plusieurs anticorps polyréactifs ou fragments de tels anticorps, caractérisée en ce que lesdits anticorps ou fragments d'anticorps présentent la capacité de lier l'ADN. Plus préférentiellement, les anticorps utilisés présentent également la capacité de lier une protéine ou un peptide du virus de l'immunodéficience acquise humaine. L'invention peut être utilisée plus particulièrement pour inhiber l'infection et/ou la réplication de différentes souches du VIH, et notamment du VIH-1 et VIH-2.

L'invention concerne plus particulièrement l'utilisation d'un ou plusieurs polypeptides tels que décrits ci-avant pour la préparation d'une composition destinée à inhiber l'infection et/ou la réplication virale.

On entend au sens de l'invention par cellule cible toute cellule naturellement susceptible d'être infectée par un virus, de préférence susceptible de permettre la réplication du virus. Ainsi, pour le VIH, il s'agit plus particulièrement des cellules du système immunitaire, et notamment de cellules lymphocytaires. On peut citer à titre de cellules cibles plus spécifiques du VIH les lymphocytes T, notamment les lymphocytes T auxiliaires (CD4+). D'autres cellules cibles du VIH au sens de l'invention sont plus généralement constituées par exemple par les cellules mononucléées périphériques, notamment humaines (PBMC). Concernant le poliovirus, on peut citer comme exemple de cellules cibles les cellules épithéliales. D'une manière générale, la présente invention peut être mise en oeuvre pour interférer avec le développement d'un virus dans tout type de cellule cible des anticorps utilisés.

Avantageusement, un polypeptide selon l'invention est dérivé de fragments scFv recombinants capables de réagir avec l'ADN ou avec d'autres macromolécules anioniques ou cationiques, en particulier l'héparine et l'héparine sulfate, et obtenus à partir de lymphocytes provenant de patients normaux ou atteints de différentes maladies notamment le lupus erythrémateux disséminé.

Le ou les mécanismes d'action des composés selon l'invention reste toujours à préciser. A cet égard, une série de résultats récemment obtenus, en particulier les virus de l'Herpès, notamment les virus de l'herpès humain par exemple le virus Herpès Simplex type 1 ou le cytomégalovirus (CMV) semble indiquer que les peptides de l'invention se fixent sur des récepteurs cellulaires utilisés par les virus eux-mêmes pour leur pénétration dans les cellules hôtes. La fixation des peptides sur ces récepteurs est suivie d'une internalisation des complexes peptides-récepteurs, ce qui se traduirait par une diminution du nombre des récepteurs cellulaires restant disponibles pour la fixation du virus. Cette diminution parfois semble être particulièrement importante comme dans le cas du CMV. Cependant, d'autres mécanismes, notamment au niveau nucléaire, pourraient également être impliqués dans les propriétés inattendues de ces anticorps et dérivés.

Avantageusement, l'activité antivirale des polypeptides s'entend au sens de la présente invention comme diminution significative de la réplication ou de l'infection. Avantageusement, l'inhibition produite par les polypeptides ou anticorps de l'invention correspond à une diminution d'un facteur 1,5 au moins par rapport au niveau d'infection et/ou de réplication dans les mêmes cellules ou populations cellulaires en l'absence de traitement. Plus préférentiellement, l'inhibition correspond à une diminution d'un facteur 4 au moins. Cette inhibition peut être quantifiée par exemple par mesure des plages virales, des niveaux d'antigènes viraux présents dans les cellules, de la viabilité cellulaire, etc. Dans un mode de mise en oeuvre particulier, l'efficacité de l'inhibition est évaluée par mesure des niveaux d'antigènes viraux tels que par exemple les antigènes p24 et/ gp120, pour le VIH.

Selon un mode de réalisation particulier, l'invention concerne l'utilisation de polypeptides pour induire une inhibition d'un facteur 2 au moins de la réplication virale dans les cellules cibles.

Dans un premier mode de mise en oeuvre, l'invention comprend l'utilisation d'un seul type de polypeptide ou fragment d'anticorps tel que défini ci-dessus. Comme illustré dans les exemples, il est en effet possible, en utilisant un seul composé, d'inhiber d'un facteur supérieur à 10, en particulier de l'ordre de 100, la réplication du VIH-1 dans les cellules cibles. L'utilisation d'un seul composé permet également l'inhibition d'un facteur supérieur à 2 de la réplication du poliovirus type-1 dans les cellules cibles.

D'autre part, une action anti-herpétique prononcée de certains peptides selon l'invention a été observée. Il est intéressant de signaler que cette action s'exerce aussi sur les HSV-1 thymidine-kinase⁻ (TK⁻), sur lesquels l'Acyclovir n'a pas d'effet.

Par ailleurs, une action prononcée anti-CMV a également été observée, notamment avec le polypeptide K19-pF4.1. Dans une série d'expériences, il a ainsi été montré que K19-pF4.1 inhibait à presque 100% l'infection des cellules par le CMV. Il est intéressant de noter que même la synthèse des antigènes précoces est complètement inhibée (détection aussi bien en immunofluorescence que par radiomarquage, suivie d'immunoprécipitation).

Dans un autre mode de mise en oeuvre, l'invention comprend l'utilisation de plusieurs polypeptides, ou fragments d'anticorps tels que définis ci-dessus. Comme illustré dans les exemples, certains de ces composés, en combinaison, permettent d'exercer un effet d'inhibition synergique due la réplication virale dans les cellules cibles. Ainsi, de manière inattendue, certains anticorps, seuls, possèdent une activité inhibitrice modérée mais, en combinaison, induisent une inhibition d'un facteur supérieur à 10, en particulier due l'ordre de 100, de la réplication du VIH-1 dans les cellules civiles.

Dans un autre mode de mise en oeuvre, l'invention comprend également l'utilisation d'un ou plusieurs polypeptides, ou fragments d'anticorps tels que définis ci-dessus en combinaison avec un ou plusieurs agents antiviraux.. De tels agents antiviraux sont par exemple l'AZT, la DDI, les antiprotéases. A cet égard; la présente demande concerne également un produit comprenant :

un ou plusieurs polypeptides, ou fragments d'anticorps tels que définis ci-dessus, et
- un agent antiviral,
en vue d'une utilisation simultanée, séparée ou espacée dans le temps.

La présente invention peut être utilisée pour inhiber l'infection et/ou la réplication virale in vitro, ex vivo ou in vivo.

Pour une utilisation in vitro ou ex vivo; les cellules cibles, ou une composition cellulaire comprenant des cellules cibles, sont généralement incubées en présence des composés tels que définis ci-dessus. Les doses de polypeptides, ou fragments d'anticorps utilisées sont généralement comprises entre 1 et 500 $\mu$g pour $10^6$ cellules environ, de préférence de 1 à 100 $\mu$g/$10^6$ cellules environ. Ces doses peuvent bien entendu être adaptées par l'homme du métier sans difficultés. L'incubation est réalisée dans tout milieu approprié à la culture des cellules, et à des conditions de température habituelles (par exemple entre la température ambiante et 37°C environ). Les milieux utilisés sont tous milieux de culture de cellules de mammifères connus de l'homme du métier, tels que par exemple les milieux RPMI, DMEM, MEM, etc. L'incubation peut être réalisée dans tout dispositif approprié, tel que boîte, flasque, ampoule, poche, flacon, seringue, etc., de préférence en conditions stériles. Avantageusement, l'incubation est réalisée pendant une période comprise entre 1 heure environ et 5 jours environ, selon l'utilisation et le but poursuivis. Il peut être envisagé par exemple d'incuber les cellules pendant une période comprise entre 1 heure et 12 heures environ. Ensuite, les cellules ainsi incubées peuvent être administrées à un sujet (autologue), et le sujet peut également recevoir une ou plusieurs administrations de fragments d'anticorps.

Pour une utilisation in vivo, les composés peuvent être administrés par différentes voies, telles que systémique, intramusculaire ou sous-cutanée par exemple. Des voies préférées sont la voie systémique (i.v. notamment) et sous-cutanée. Les doses utilisées peuvent également être adaptées par l'homme du métier en fonction du stade du sujet, du but poursuivi et du nombre et/ou de la fréquence des administrations.

Préférentiellement, l'invention est utilisée pour inhiber la réplication ou l'infection virale dans des cellules cibles ex vivo. A cet effet, les cellules cibles sont prélevées chez un sujet (par exemple les PBMC), incubées ex vivo avec des composés tels que définis ci-avant (par exemple pendant 1 à 6 heures, à 37°C, dans une poche stérile), puis réadministrées au sujet. Le sujet peut également recevoir une ou plusieurs administrations des composés, éventuellement en combinaison avec un ou plusieurs autres agents antiviraux. Les composés ou compositions tels que décrits ci-dessus sont particulièrement adaptés à une utilisation à titre préventif, c'est-à-dire pour inhiber l'infection ou la réplication virales chez des sujets sains ou séropositifs mais n'ayant pas développé les symptômes de la maladie. L'invention peut également être utilisée comme traitement de maintien, seule ou en combinaison avec d'autres agents antiviraux, comme expliqué ci-avant.

A cet égard, l'invention concerne également une méthode permettant d'améliorer l'efficacité des agents antiviraux comprenant l'utilisation combinée de polypeptide, ou fragments d'anticorps tels que définis ci-avant.

L'invention concerne également un procédé de modification d'une cellule dans le but de diminuer l'infection de cette cellule par un virus et/ou la réplication d'un virus dans cette cellule (i.e., pour améliorer la résistance virale de cette cellule), comprenant la mise en contact de cette cellule avec un ou plusieurs polypeptides, ou fragments d'anticorps tels que définis ci-avant. L'invention concerne également toute population de cellules incubée en présence de polypeptides, ou fragments d'anticorps tels que définis ci-avant. De telles populations peuvent être plus particulièrement des cellules PBMC ou d'autres cellules du système immunitaire, éventuellement conditionnées dans un dispositif stérile. Préférentiellement, une telle composition cellulaire comprend généralement de $10^5$ à $10^8$ cellules. Ces compositions cellulaires peuvent être utilisées pour l'étude du cycle viral, la recherche de composés inhibiteurs ou d'associations de composés inhibiteurs, ou éventuellement pour diminuer les risques et les effets d'une infection virale in vivo après administration.

L'invention est aussi relative à une composition pharmaceutique comprenant, en association avec un véhicule physiologiquement acceptable, un vecteur tel que défini ci-avant dans lequel la substance est un principe actif de médicament.

L'invention concerne encore un vaccin comprenant, en association avec un véhicule physiologiquement acceptable, un vecteur tel que défini ci-avant dans lequel la substance est un antigène.

## Légende des Figures

Figure 1 : Représentation de la structure d'un anticorps.

Figure 2 : Séquence peptidique des régions variables de la chaîne lourde d'anticorps monoclonaux pénétrants. Les régions CDR sont représentées. Les "-" représentent des acides aminés identiques.

Figure 3 : Séquence des régions CDR2 et CDR3 des domaines VH d'anticorps anti-ADN pénétrants dans les cellules.

Figure 4 : Mise en évidence, par ELISA, de l'effet adjuvant in vivo des polypeptides de l'invention.

Figure 5 : Activité de transfection d'ADN du peptide CDR2-3-PL19 dans les cellules CCL39.

Figure 6 : Polyfection de cellules 3T3 par le peptide K19-P3 à différents rapports peptide/plasmide.

Figure 7 : Polyfection de cellules 3T3 par le peptide K19-P3 en fonction du temps d'incubation des cellules avec le complexe.

Figure 8 : Polyfection de cellules 3T3 en l'absence (Figure 8a) ou en présence (Figure 8b) d'un agent facilitateur : le glycérol.

Figure 9 : Polyfection de cellules CCL39 en l'absence (Figure 9a) ou en présence (Figure 9b) d'un agent facilitateur : le glycérol.

Figure 10 : Titres infectieux et dosage de l'antigène p24 du $VIH_1$ produit dans le milieu de culture de cellules mononuclées circulantes humaines (PBMC) traitées par différentes préparations d'anticorps et infectées par $VIH_1$ Lai. Valeurs moyennes de trois expériences indépendantes.

Figure 11 : Titres infectieux et dosage de l'antigène p24 du $VIH_1$ produit dans le milieu de culture des PBMC humains traités par différentes préparations de polypeptides ou d'anticorps et infectées par $VIH_1$ souche BX 08.

11A et 11B : Mesure des titres infectieux au jour 13 dans deux séries d'expériences indépendantes (Expériences III et V).

11C : Quantification de l'antigène p24 dans les surnageants des cultures inoculées par la dilution $10^{-2}$ au jour 9. (Expérience V).

Figure 12 : Inhibition de la réplication du poliovirus par des polypeptides par mesure du facteur de diminution du titre viral au jour 5.

Barres remplies : souche de poliovirus sauvage ;
Barres hachurées : souche de poliovirus atténuée.

Figure 13 : Mise en évidence de l'inhibition de la synthèse des protéines du CMV après traitement avec le polypeptide K19-pF4.1 (PL), le peptide P3, ou aucun peptide (NT), avant l'infection virale (13a, 13b), pendant l'infection virale (13c) ou après l'infection virale (13d). La révélation est effectuée au moyen d'un anticorps anti-pp150 (pour les protéines tardives), anti-pp65 (pour les protéines précoces) et anti-IE (pour les protéines très précoces).

Matériel et méthode.

Souris et lignée cellulaires :

Des souris BALB/c (NZB x NZW) F1 ont été maintenues dans l'animalerie de l'Institut Pasteur. Les cellules suivantes d'espèces différentes et de tissus différents sont utilisées : cellules PtK2 (Fibroblastes de rein), cellules GMA-32 (poumon de hamster), cellules 3T3 (Fibroblastes d'embryon de souris) cellules CCL39 (fibroblastes de hamster), cellules HeLa (Carcinome Cervix humain), cellules VERO (rein de singe), cellules HEp-2 (Carcinome de larynx humain), cellules JURKAT et cellules CEM (lymphoblastes T humain) toutes disponibles à la Collection ATCC. Ces différents types cellulaires ont été cultivés en milieu RPMI ou en milieu DMEM contenant 10 % de sérum de veau foetal inactivé et supplémenté par de la L-glutamine, du pyruvate de sodium et des acides aminés non-essentiels ainsi que des antibiotiques (milieu de culture complet) à 37°C dans une atmosphère humidifiée à 5 % de $CO_2$.

Anticorps monoclonaux

La préparation et l'isolement des anticorps monoclonaux J20.8, F4.1 et F14.6 ont été décrits dans la demande de brevet FR N° 9508316. Ces anticorps sont des anticorps polyréactifs anti-ADN et reconnaissant également différents antigènes tels que notamment les peptides 22-37 et 46-60 de la protéine Tat. Ces anticorps sont des IgG2a murines. L'anticorps anti-TAT utilisé est un anticorps monoclonal murin IgG1 monoréactif reconnaissant la protéine TAT du VIH-1.
Ces anticorps ont été purifiés sur colonne de protéine A Sépharose (Ey et al., Immunochemistry 15 (1978) 429). La polyréactivité de ces anticorps purifiés, vis-à-vis de l'ADN double-brin d'une part et d'autres antigènes d'autre part a été testée par ELISA en utilisant des méthodologies décrites dans la littérature (Guilbert et al., J. Immunol. 128 (1982) 2779).

Synthèse Peptidique

Les synthèses peptidiques ont été réalisées selon les techniques connues de l'homme du métier. Ainsi, les peptides sont produits par synthèse en phase solide sur résine Fmoc. Le clivage est réalisé par l'acide trifluoroacétique (TFA) et les peptides ont été purifiés sur colonne HPLC-RP C5 semi-préparative (Eurosil Bioselect 5μ, 300 A (1;6 x 25 cm)) et élués à 1,1 ml/mn avec une solution 0,1% TFA et un gradient d'acétonitrile (10-70%). Les peptides lyophilisés ont été dissous dans du NaCl 0,15 mM et stérilisés sur filtre 0,22 μm. Pour déterminer la concentration en peptide, des aliquots ont été hydrolysés à 110°C en présence d'HCl 6N - 2% phénol puis analysés sur un analyseur d'acides aminés Beckman 6300.

Souches virales

Les expériences décrites ci-après ont été réalisées en utilisant les souches virales suivantes : VIH-1 souche BX 08 (isolat primaire de sous-type B) ; VIH-1 souche Lai ; .poliovirus type 1 (souche sauvage PV1 Mahoney et souche atténuée PV1 Sabin) ; cytomégalovirus souche Ad169 et Herpès Virus Simplex de type I.

**Exemples**

1. Séquençage des anticorps monoclonaux

La séquence nucléotidique des régions VH et VL des anticorps monoclonaux J20.8, F4.1 et F14.6 a été déterminée. Pour cela, l'ARN total a été extrait des cellules d'hybridomes en utilisant la technique de thiocyanate de guanidine (Schwartz et al., Biol. Cell: 73 (1991) 7) puis séparé par électrophorèse sur un gel de formaldehyde/Agarose.

Les ARN messagers ainsi obtenus ont ensuite été transformés en ADN complémentaires en utilisant un kit de transcriptase reverse (Life Technologies, Eràgny, France) et utilisés comme amorce dans des réactions d'amplification (PCR) en utilisant l'ADN polymérase Taq (Boehringer, Mannheim, FRG) en suivant les recommandations du fabricant. Les amorces oligonucléotidiques utilisées pour générer les ADN complémentaires sont :

- d'une part une amorce correspondant aux séquences conservées des immunoglobulines IgG2a:

    5'-GTTCTGACTAGTGGGCACTCTGGGCT (SEQ ID n° 11)

- et d'autre part, quatre amorces pour la région VH :

    5'-GAGGTTCAGCTCGAGCAGTCTGGGGC (SEQ ID n° 12)
    5'-GAGGTGAAGCTCGAGGAATCTGGAGG (SEQ ID n° 13)
    5'-GAAGTGCAGCTCGAGGAGTCTGGGG (SEQ ID n° 14)
    5'- GAGGTTCAGCTCGAGCAGTCTGGAGC (SEQ ID n° 15)

Les produits d'amplification par PCR ont ensuite été purifiés en utilisant le kit Geneclean (Bio 101, Vista, CA). Le séquençage chimique a été réalisé par Genome express (Grenoble, France). Les séquences nucléotidiques ont été analysées en utilisant les banques de données GENBANK et EMBL conservées à L'Institut Pasteur (Unité d'Informatique Scientifique) en utilisant le logiciel d'analyse des séquences (GCG) (Devereux J., The GCG Séquence Analysis Software Package, 1989), et les séquences d'acides aminés correspondantes ont été déduites.

La séquence des régions VH de ces anticorps est représentée sur la Figure 2. L'alignement de ces séquences permet de localiser les régions CDR présentes dans ces séquences (CDR1, CDR2 et CDR3). Cet alignement permet en outre de mettre en évidence l'existence d'une homologie structurale importante entre les régions CDR2 et de caractéristiques structurales communes entre les régions CDR3, notamment la présence de résidus basiques (arginine et lysine). Les séquences de régions CDR2 et CDR3 d'autres anticorps sont présentées sur la figure 3.

### 2. Construction de polypeptides pénétrants

A partir des séquences présentées sur la figure 1, différents polypeptides comportant tout ou une partie de la région CDR3 et/ou de la région CDR2 des anticorps ont été préparés. La synthèse a été réalisée par des synthétiseurs de peptides (Cf matériel et méthodes). Les polypeptides suivants sont synthétisés, dans lesquels m vaut 1 ou 0 :

### CDR3 :

SEQ ID N° 1 :

    $(Thr)_m$-$(Arg)_m$-$(Gln)_m$-Lys-Tyr-Asn-Lys-Arg-Ala-(M-D-Y-W-G-Q-G-T)m

Une variante de cette séquence est par exemple la séquence TRQKYNKRA(MDYWGQGT)m. Une autre variante (homologue fonctionnel) est par exemple la séquence .Ala-Arg-Gln-Lys-Tyr-Asn-Lys-Arg-Ala-Met-Asp-Tyr (SEQ ID n° 8).
SEQ ID n° 2 :

    $(Thr)_m$-$(Arg)_m$-$(Gln)_m$-Lys-Tyr-Gly-Lys-Arg-Gly-(M-D-Y-W-G-Q-G-T)m

Une variante de cette séquence est par exemple la séquence TROKYNKKRG(MDYWGQGT)m.
SEQ ID n° 3 :

    $(Thr)_m$-$(Arg)_m$-$(Gln)_m$-Ala-Arg-Ala-Thr-Trp-Asp-Trp-(F-A-Y-W-G-Q-G-T)m

Une variante de cette séquence est par exemple la séquence TRGARATWDW(FAYWGQGT)m.
Dans les séquences 1-3 ci-dessus, MDYWGQGT = Met-Asp-Tyr-Trp-Gly-Gln-Gly-Thr et FAYWGQGT = Phe-Ala-Tyr-Trp-Gly-Gln-Gly-Thr. En outre, la formule (a-b-c)m signifie que un seul, plusieurs ou tous les résidus mentionnés dans la parenthèse sont présents ou non.

### CDR2 :

SEQ ID n° 4 :

(Val)$_m$-(Ala)$_m$-Tyr-Ile-Ser-Arg-Gly-Gly-Val-Ser-Thr-Tyr-Tyr-Ser-Asp-Thr-Val-Lys-Gly-(Arg)$_m$-(Phe)$_m$-(Thr)$_m$ (CDR2 (1)). Une variante de cette séquence est par exemple la séquence VAYISRGGVSTYYSDTVKGRF ou VAYISRGGVSTYYSDTVKGRFT.

SEQ ID n° 5 :

(Val)$_m$-(Ala)$_m$-Tyr-Ile-Ser-Arg-Gly-Gly-Gly-Ile-Phe-Tyr-Tyr-Gln-Asp-Ser-Ile-Lys-Gly-(Arg)$_m$-(Phe)$_m$ (CDR2(2)). Une variante de cette séquence est par exemple la séquence VAYISRGGIFYYQDSIKGRF.

SEQ ID n° 6 :

(Val)$_m$-(Ala)$_m$-Ala-Ile-Ser-Arg-Gly-Gly-Gly-Tyr-Ser-Tyr-Tyr-Leu-Asp-SerVal-Lys-Gly-(Arg)$_m$-(Phe)$_m$-(Thr)$_m$-(Ile)$_m$ (CDR2(3)). Une variante de cette séquence est par exemple la séquence VAAISRGGGYSYYLDSVKGRFTI

CDR2-3 :

SEQ ID n° 7 (peptide P3 ou pF4.1):

Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala. Une variante de cette séquence est par exemple la séquence VAYISRGGVSTYYSDTVKGRFTR-QKYNKRAVAY.

CDR2-3 fonctionnalisé :

Biotinyl-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala-

(correspondant à la séquence SEQ ID n° 7)

Cys-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala- (SEQ ID n° 9).

Dans la séquence SEQ ID n° 9, on introduit par la cystéine un groupement actif (SH), pour faire le couplage à une autre substance.

CDR2 fonctionnalisé :

Biotinyl-VAYISRGGVSTYYSDTVKGRFT (Biotinyl-Val-Ala-Tyr-Ile-Ser-Arg-Gly-Gly-Val-SerThr-Tyr-Tyr-Ser-Asp-Thr-Val-Lys-Gly-Arg-Phe-Thr), correspondant à la séquence SEQ ID n° 4.

CDR3 fonctionnalisé :

Biotinyl-Ala-Arg-Gln-Lys-Tyr-Asn-Lys-Arg-Ala-Met-Asp-Tyr (correspondant à la séquence SEQ ID n° 8).

3. Étude de la Pénétration des polypeptides dans les cellules

Des fibroblastes PtK2 en culture, ensemencées la veille à raison de 5 x 10$^4$ cellules par puits sur des lamelles de verre, sont incubées à 37°C, 1-18 heures dans du milieu de culture RPMI 1640 (ou DMEM) complet (10% de sérum de veau foetal, 2 mM L-glutamine et 1 mM pyruvate de sodium) contenant un polypeptide de l'invention biotinylé (5-20 µg/ml). Les cellules sont ensuite lavées avec du PBS et fixées avec 2% de p-formaldehyde à 4°C pendant 10 minutes puis lavées avec du PBS.
Les cellules sont ensuite incubées avec une solution de streptavidine conjuguée à la peroxydase à 5 µg/ml dans le PBS pendant 30 minutes, puis lavées au PBS et incubées dans le substrat cytochimique de la peroxydase (diaminobenzidine + $H_2O_2$). Après lavage, les cellules sont examinées au microscope.

Les résultats obtenus montrent que, après 1 heure de culture, les polypeptides comportant tout ou partie d'un CDR3 sont visibles par la coloration de la peroxydase dans le cytoplasme de toutes les cellules et dans le noyau d'un grand nombre de cellules. Les résultats montrent en outre que le polypeptide CDR2-3 (notamment le polypeptide pF4.1 de séquence SEQ ID n° 7) pénètre massivement et rapidement dans les cellules, et atteint en majorité le noyau desdites cellules. Ces résultats montrent donc qu'il est possible, à partir d'un fragment de type CDR3, de générer des polypeptides ayant une capacité élevée de pénétration cellulaire.

4 Pénétration des vecteurs polypeptide-streptavidine couplée aux enzymes dans les cellules.

Cet exemple illustre comment les polypeptides de l'invention peuvent être couplés à une substance active, et utilisés pour véhiculer ladite substance dans les cellules.

Les vecteurs sont préparés en incubant 1,4 $\mu$g de polypeptide CDR2-3 (pF4.1) biotinylé avec 10 $\mu$g de strep-tavidine conjuguée à la peroxydase ou à la phosphatase alcaline dans un volume de 10 $\mu$l pendant 15 minutes à la température du laboratoire. Le mélange est ensuite dilué dans 0.5 ml de culture complet avant d'être déposé sur les cellules en culture. Après 2 heures de culture, les cellules sont lavées au PBS, fixées au p-paraformaldehyde, lavées puis incubées dans le substrat cytochimique de la peroxydase (diaminobenzidine + $H_2CO_2$) ou celui de la phosphatase alcaline (Naphtol AsMx + le sel de tetrazolium Fast Red).

Les résultats obtenus montrent que l'on détecte les enzymes correspondants au niveau du cytoplasme de toutes les cellules de la culture et faiblement jusqu'à intensément dans la majorité des noyaux de ces cellules. En revanche, aucune coloration intracellulaire n'est observée quand les cellules sont incubées en présence de streptavidine couplée à la peroxydase, streptavidine couplée à la phosphatase alcaline ou avec la streptavidine ou les enzymes sous leurs formes natives.

5. Construction et activité d'un polypeptide comportant des résidus lysine supplémentaires.

Un polypeptide CDR2-3-PL19 (polylysine) (également désigné K19-P3 ou K19-pF4.1) a été synthétisé et purifié (ALTERGEN). La séquence de ce polypeptide est la suivante :

(NH2-(K19)-VAYISRGGVSTYYSDTVKGRFTRQKYNKRA-COOH) SEQ ID n° 10.

Les cellules CCL39 (fibroblastes de Hamster) (5 x $10^4$) cellules) sont mises dans des plaques de cultures 24 puits 18 heures avant la transfection dans du milieu de culture MEM + 10% SVF (sérum de veau foetal). Les transfections sont réalisées dans du MEM + 10% SVF sans aucun autre agent auxilliaire (CHLOROQUINE). Le peptide-PL et la polylysine libre PL (correspondant à 19 lysines) sont complexés avec le plasmide pCMVLUC (respectivement à 24 $\mu$g et 70 $\mu$g pour 6 $\mu$g de plasmide) pendant 30 minutes. Puis le complexe est ajouté goutte à goutte sur les CCL39. Après 5 heures d'incubation, le milieu est remplacé par du milieu frais. L'expression de la luciférase est déterminée 24 heures après. Les cellules sont lavées 2 fois avec du PBS. Après le lavage les cellules sont lysées avec 100 $\mu$l de tampon de lyse (PROMEGA) pendant 10-15 minutes. Les cellules sont ensuite centrifugées 7 minutes à 4°C pour enlever les débris cellulaires. 20 $\mu$l de ce_ lysat est mélangé avec 100 $\mu$l du tampon luciférase (PROMEGA). Les unités relatives de la luciférase (RLU) sont enregistrées sur un LUMAT LB9501 (BERTHOLD). La concentration des protéines est déterminée par le Kit BIORAD PROTEIN ASSAY-1 et le taux de luciférase dans chaque échantillon est normalisé par mg de protéine, chaque transfection étant réalisée trois fois.

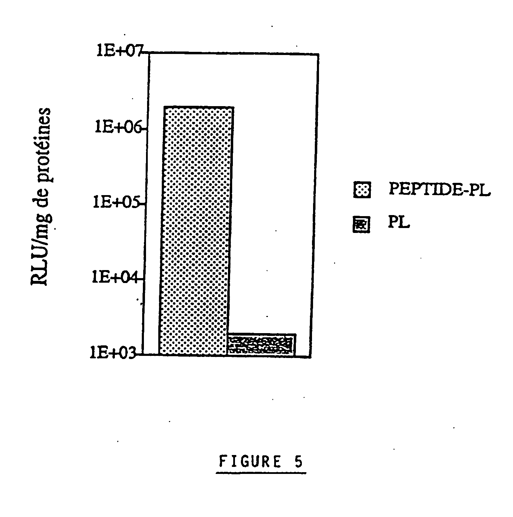

Les résultats obtenus sont présentés sur la Figure 5. Ils montrent que le peptide-PL transfecte en milieu complet et sans aucun agent auxiliaire avec une efficacité de 2 x $10^6$ RLU/mg de protéines soit environ 1000 fois plus que la polylysine seule et autant qu'un peptide récemment décrit (Wadhwa et al., Bioconjugate Chen. 1997, 8:81-88.), mais dont l'activité est dépendante de la présence de 100$\mu$M de chloroquine.

La transfection des cellules par le polypeptide CDRK19-P3 est donc particulièrement avantageuse puisqu'elle peut être réalisée dans un milieu de culture complet et en absence d'agent auxiliaire. Actuellement les systèmes de transfection utilisant la polylysine nécessitent tous l'addition d'agent auxiliaire, le plus souvent la chloroquine qui est toxique pour les cellules. Cette chloroquine permet d'éviter la dégradation dans les lysosomes des complexes conjugués-polylysine intemalisés par la voie classique d'endocytose.

La présente invention, au contraire, ne nécessite pas l'emploi de tels agents auxiliaires.

6. Polyfection de cellules 3T3 avec le peptide K19-P3

Cet exemple illustre les propriétés de transfert d'acides nucléiques des peptides de l'invention dans les cellules 3T3.

Les cellules 3T3 (8 x 10$^4$ cellules) ont été étalées dans des plaques de_ 24 puits le jour précédent la transfection. Les polyplexes entre le plasmide pCMV LUC et le peptide K19-P3 ou les peptides contrôles CW-K19 et K19 ont été préparés par incubation 20 minutes à 20°C de 3μg de plasmide dans 50 μl de NaCl 0,15 M, et de quantités variables de peptide. Plus particulièrement, les polyplexes ont été réalisés à des stoechiométries allant de 0,05 à 1,4 nmoles de peptides par μg d'ADN.

Peu avant la transfection, le cellules ont été lavées, puis incubées pour des périodes variables avec 0,5 ml de milieu de culture complet. Les polyplexes ont été ajoutés aux cellules pendant 1 h 15, 2 h 30, 5 h 30 et 24 h à 37°C dans une atmosphère humidifiée (92% air, 8% $CO_2$). Le milieu a été éliminé et les cellules incubées à nouveau pendant 24 heures à 37°C dans 1 ml de milieu frais. Chaque expérience a été réalisée au moins en triple. L'activité luciférase a été déterminée comme dans l'exemple 5.

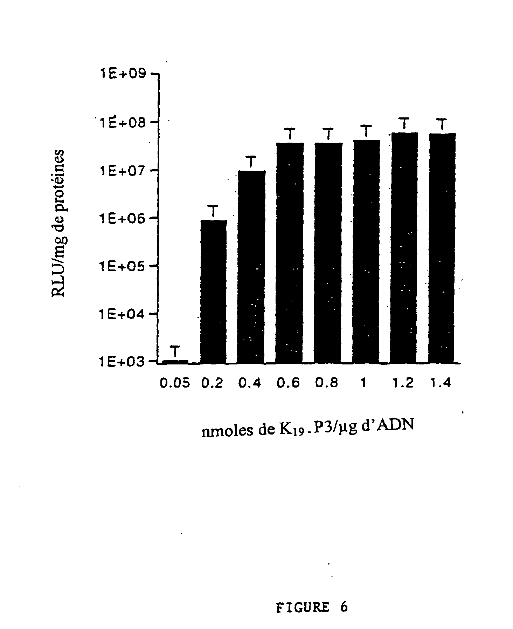

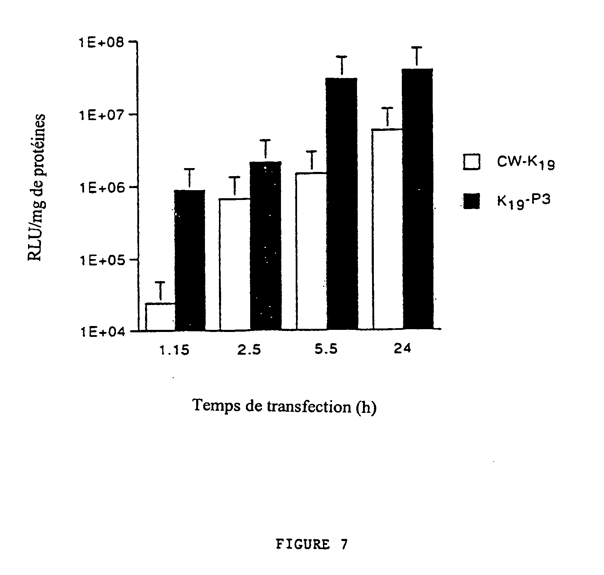

Les résultats obtenus sont présentés sur les figures 6 et 7.

La figure 6 montre une augmentation de l'expression de la luciférase corrélée à l'augmentation de la concentration en peptide, l'activité maximum étant observée à une concentration de 0,6 nmole de peptide/μg d'ADN. Cette concentration correspond à un rapport de charges R = 4,4. Aux concentrations plus élevées, l'expression demeure constante.

La figure 7 montre par ailleurs que, à un rapport de charges R = 4,4, l'exposition des cellules pendant 24 heures au polyplexe n'induit aucune toxicité. Après 5 h 30 d'incubation, l'activité luciférase mesurée est de 2-3 x 10$^7$ RLU /mg de protéine pour le peptide K19-P3. La transfection après 24 h d'incubation est augmentée d'un facteur 1,3 environ par rapport à une incubation de 5 h 30.

### 7. Polyfection en présence de glycérol

Cet exemple montre que l'efficacité de transfection peut être améliorée en utilisant une composition comprenant un peptide de l'invention et un stabilisant, tel le glycérol.

Dans cet exemple, les transfections ont été réalisées sur cellules 3T3 et CCL39, comme décrit dans l'exemple 6, dans un milieu complet contenant ou non du glycérol (0,23 M), à un rapport de charge peptide/ADN de 2.2 (incubation 5 h 30 à 37°C).

Par ailleurs, à titre comparatif, une transfection a été effectuée en présence de polyéthylène immine (PEI) 25 kDa (Aldrich, St Louis, MO), à un rapport de charge de 2,X2 en présence de glycérol et de 5 en absence de glycérol.

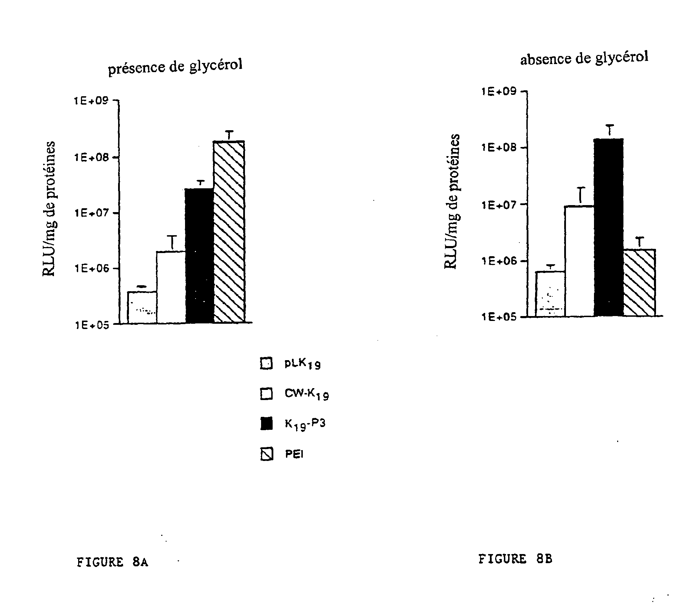

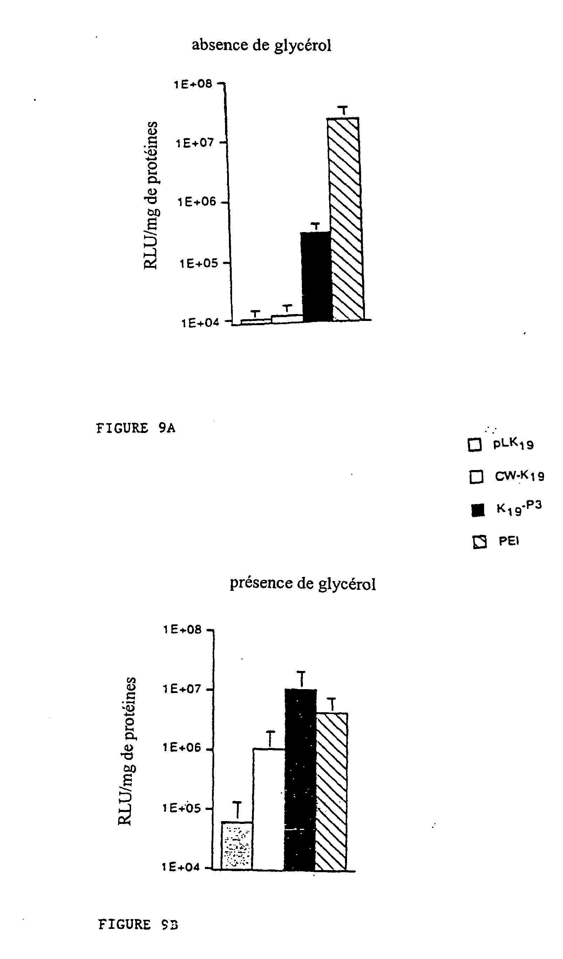

Les résultats obtenus sont présentés sur les figures 8 et 9.

Ces résultats montrent que le glycérol (0,23 M) induit une augmentation de la polyfection (déterminée par mesure de l'activité luciférase) d'un facteur supérieur à 5, et pouvant atteindre 40. Ainsi, dans les cellules CCL39 (figure 9), une augmentation d'un facteur 38 à été mise en évidence, ce qui illustre l'intérêt d'une composition de l'invention comprenant du glycérol, notamment pour la transfection de cellules difficiles à transfecter. Par ailleurs, des résultats similaires ont été obtenus en faisant varier la concentration en glycérol du milieu, jusqu'à un niveau de 1,15M.

Les résultats présentés sur les figures 8 et 9 montrent également que les peptides de l'invention, qui sont non-toxiques et de structure simple et définie, présentent une efficacité de transfection supérieure au PEI 25kDa en présence de glycérol, à la fois sur les cellules 3T3 et CCL39.

### 8. Utilisation d'un polypeptide comme immunoadjuvant

Le vecteur est formé en laissant en incubation pendant 15 minutes 14 μg de polypeptide CDR2-3 biotinylé + 40 μg de streptavidine conjuguée à la peroxydase (Sigma) puis dilué dans 0.1 ml de PBS avant d'être injecté à chaque souris. L'injection se fait dans les coussinets plantaires. Les souris témoins reçoivent 40 μg de streptavidine conjuguée à la peroxydase dans 0.1 ml de PBS.

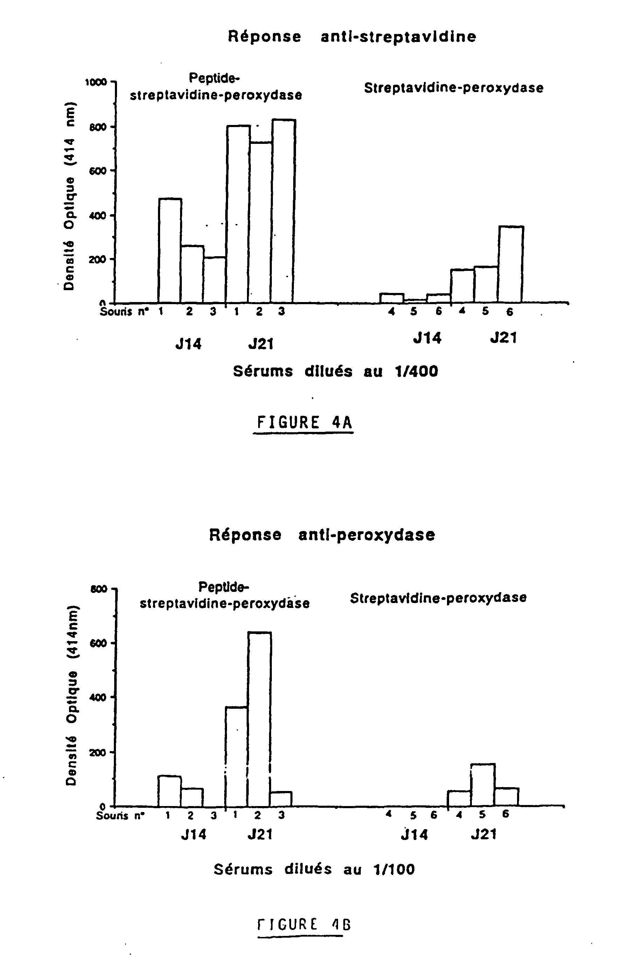

Les souris sont saignées toutes les semaines. Une injection de rappel est effectuée dans les mêmes conditions un mois après la première injection. Par un test ELISA, on observe que les souris ayant reçu le complexe CDR2-3-streptavidine conjuguée à la peroxydase répondent en anticorps IgG anti-streptàvidine et anti-peroxydase, mais très peu en IgM, avec des valeurs nettement plus élevées que celles qui ont reçu la streptavidine conjuguée à la peroxydase seule et ceci dès le 14ème jour (Figure 4). L'injection de rappel entraîne une augmentation du titre en anticorps dans les deux groupes, mais les valeurs sont toujours supérieures dans le groupe ayant reçu le complexe. Sur la base de ces résultats, il apparaît donc que dans les conditions testées, les polypeptides de l'invention sont capables d'augmenter, d'un facteur 4 à 8 au moins, le titre des anticorps dirigés contre un antigène donné.

Les mêmes expériences peuvent être reproduites en utilisant comme antigène non plus une protéine, mais un acide nucléique codant pour ledit antigène. Par ailleurs, ces expériences peuvent également être répétées dans les mêmes conditions avec un polypeptide comportant des résidus basiques supplémentaires, notamment une polylysine.

9. Inhibition du VIH

Cet exemple illustre les propriétés antivirales des anticorps polyréactifs selon l'invention sur le VIH-1, souche Lai.

Cellules

Les cellules cibles du VIH utilisées sont des cellules mononucléées du sang périphérique (PBMC) humain. Ces cellules sont obtenues à partir de sujets sains par toute technique connue de l'homme du métier (gradients Ficoll, leukaphérèse, etc). Les PBMC sont activés par la phytohémagglutinine pendant 3 jours environ et maintenus en culture à 37°C en atmosphère $CO_2$, en présence d'interleukine 2.

Dosage de l'antigène p24

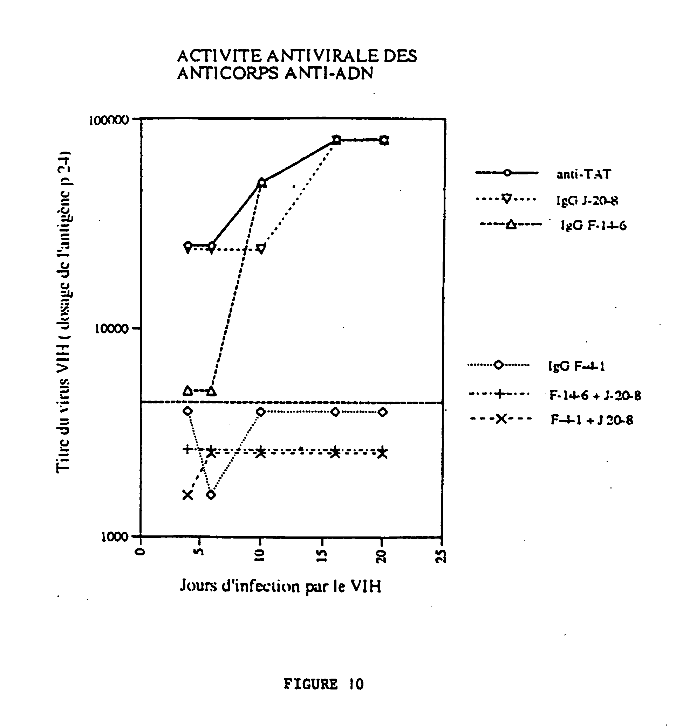

La protéine p24 a été dosée dans les sumageants de culture des cellules par test ELISA en utilisant un kit commercial (Diagnostic Pasteur).
Des cellules mononucléées périphériques humaines (PBMC), après activation par la phytohémagglutinine, ont été incubées à différentes concentrations avec les trois anticorps J20-8, F14-6 et F4-1 pendant 4 heures à 37°C. Après élimination des anticorps, les cellules ont été infectées par des dilutions successives de $VIH_1$ Lai (1 heure à 37°C), lavées, incubées avec du milieu de culture frais en présence ou non d'anticorps et les surnageant ont été examinés tous les 3 ou 4 jours pour la présence de l'antigène P24 du $VIH_1$ pour évaluer le niveau de réplication virale dans les cellules traitées ou non. Les résultats de ces titrages du $VIH_1$ Lai sur des PBMC traités ou non par les anticorps sont présentés sur la figure 10. Ces résultats montrent que l'anticorps F4-1 est capable de réduire d'environ 2 log le titre du virus comparativement aux cellules non traitées ou traitées par l'anticorps monoréactif anti-TAT. De plus, ces résultats démontrent également que l'anticorps F14-6, qui n'inhibe pas le $VIH_1$ quand il est testé seul, provoque une réduction du titre infectieux en synergie avec l'anticorps J20-8 qui seul ne possède pas d'activité inhibitrice.
Il est fort probable que l'action des anticorps selon l'invention s'exerce une fois que l'anticorps a pénétré à l'intérieur des cellules, comme illustré par le fait qu'une pré-incubation des cellules avec l'anticorps permet d'induire une inhibition forte de l'infectiosité du $VIH_1$. En outre, des expériences préliminaires indiquent que le traitement par les anticorps ne modifie pas la reconnaissance virus- cellules via la molécule CD4, ce qui suggère un effet spécifique et intracellulaire des anticorps de l'invention sur l'infectiosité et la réplication du VIH.

10. Inhibition du VIH

Cet exemple illustre à nouveau les propriétés des compositions selon l'invention, notamment des polypeptides, sur un autre isolat du VIH.
Des cellules mononucléées périphériques humaines (PBMC) sont activée en présence de phytohémagglutinine pendant 3 jours, puis resuspendues dans un milieu de culture en présence d'interleukine-2. Les cellules sont ensuite préincubées, pendant 4 heures à 37°C, avec 25 $\mu$g ou 50 $\mu$g pour $2.10^6$ cellules des polypeptides ou anticorps suivants :

- Peptide P3 : ce peptide correspond au peptide pF4.1 de séquence SEQ ID NO: 7
- Peptide P3PL : ce peptide correspond au peptide K19-pF4.1 de séquence ID NO: 10
- Anticorps F4-1.

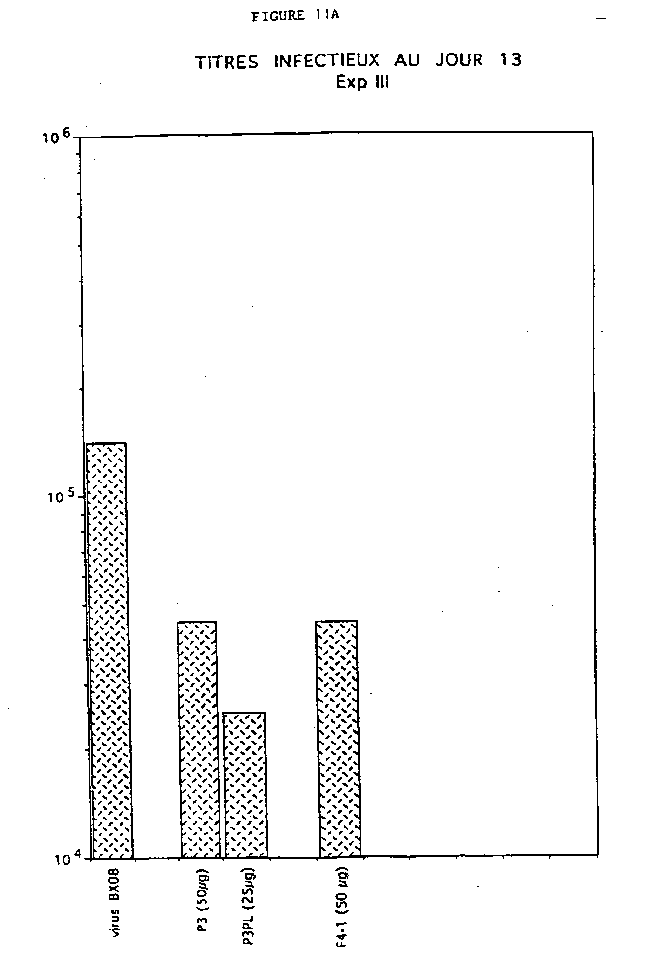

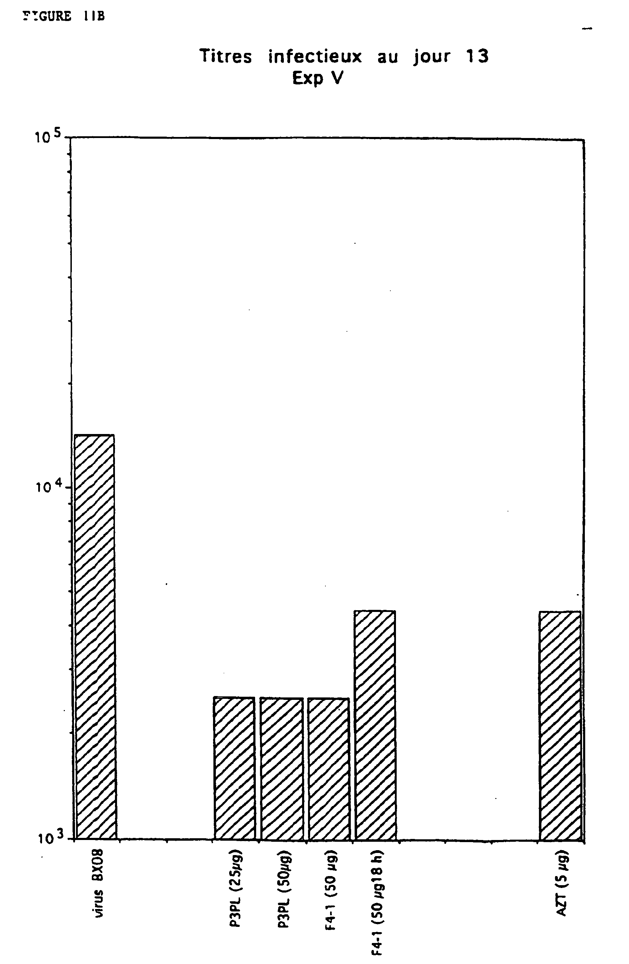

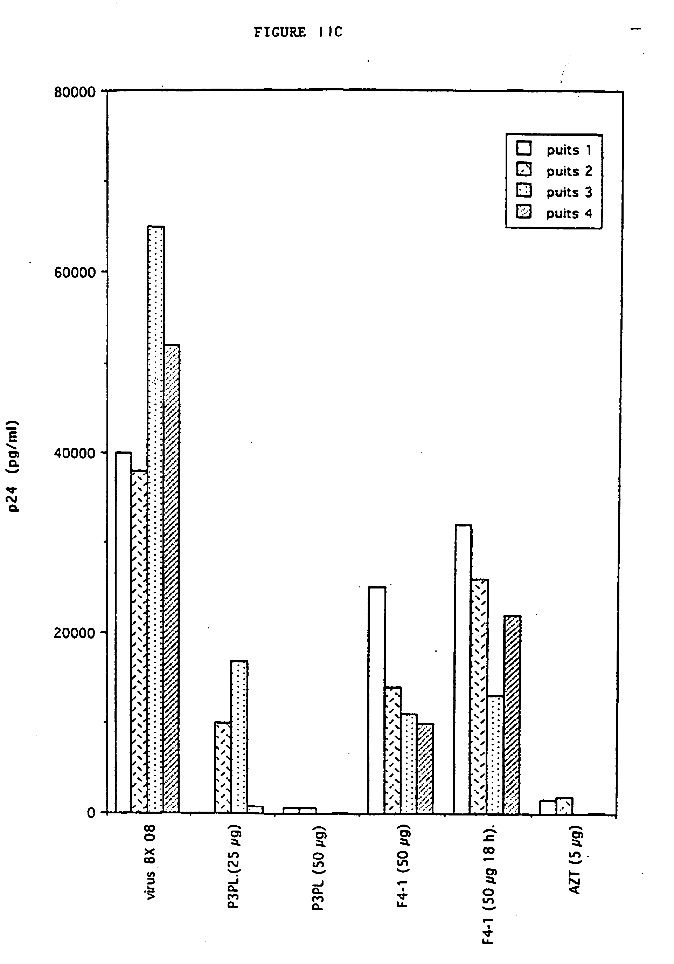

A titre comparatif, les cellules ont également été incubées en présence d'AZT.
Après incubation, les cellules sont lavées dans du milieu de culture frais puis infectées avec 0,5 ml de dilution virale de VIH-1, souche BX 08 (de $10^{-3}$ à $10^{-5}$ ou de $10^{-1}$ à $10^{-4}$ selon les expériences) sur un culot de $2.10^6$ cellules pendant 1 heure à 37°C. Les cellules sont ensuite lavées 3 fois dans du milieu frais et resuspendues dans du milieu contenant les peptides ou anticorps ci-dessus (12,5 $\mu$g ou 25 $\mu$g) et réparties dans des plaques 48 puits à raison de 4 puits par dose de virus et de peptide/anticorps. Le milieu de culture, contenant les peptides/anticorps, est changé tous les 3 à 4 jours. Ensuite, la production virale (conséquence de l'infection et de la réplication) est estimée par dosage de l'antigène p24 dans les sumageants de culture, dans les conditions décrites dans les Matériels et Méthodes.
Les résultats de plusieurs séries d'expériences sont présentés sur les figures 11A, 11B et 11C.
Comme dans l'exemple 9, ces résultats illustrent la capacité d'anticorps, polyréactifs anti-ADN ou des polypeptides de réduire la réplication du VIH dans des cellules cibles, à différentes doses.

11. Inhibition du Poliovirus

Cet exemple illustre les propriétés antivirales des produits de l'invention, sur des poliovirus.

Afin de tester le pouvoir inhibiteur des peptides K19 pF4.1, K19 pJ20.8, K19 pF14.6 sur la réplication du poliovirus, deux souches de poliovirus de type 1 (PV1) ont été utilisées : la souche sauvage PVA/Mahoney et la souche atténuée PV1/Sabin. Le pouvoir inhibiteur des peptides a été évalué en mesurant le facteur de diminution du titre d'une suspension virale de poliovirus en présence de peptide. Le titre des suspensions virales est déterminé en dose cytopathogène 50 (DCP50) par ml sur des cellules épithéliales humaines Hep-2c par une microtechnique en dilution limite (Melnick et al., 1979, Melnick, J.L., Wenner, H.A. and Philips, C.A. (1979). Enteroviruses. In "Diagnostic procedure for viral, rickettsial and chlamydial infections" (E.H. Lennette and N.J. Schmidt, Eds), pp. 471-534. American Public Health Association, Washington, D.C.). Le facteur de diminution du titre correspond donc au rapport entre le titre de la suspension virale en absence de peptide et celui en présence du peptide testé.

## Matériel

### Peptides

Peptides dérivés d'anticorps monoclonaux anti-DNA : K19 pF4.1 (SEQ ID NO:10);
K19 pFJ20.8
$(K)_{19}$-V-A-Y-I-S-R-G-G-G-I-F-Y-Y-Q-D-S-I-K-G-R-F-T-R-E-K-Y-G-K-R-G-M-D-Y,
K19 pF14.6
$(K)_{19}$-A-I-S-R-G-G-G-Y-S-Y-Y-L-D-T-V-K-R-T-A-R-A-T-W-D-W-F-A-Y.
Peptide utilisé comme contrôle négatif : K19 PT correspondant à un peptide de l'ovalbumine de 20 acides aminés portant du côté N-terminal 19 lysines.

### Virus

Souche sauvage de poliovirus de type 1 : PV1/Mahoney
Souche atténuée de poliovirus de type 1 : PV1/Sabin

### Cellules

Cellules épithéliales humaines Hep-2c ayant pour origine un carcinome- épidermoïde du larynx.

### Protocole

Au jour 1, les cellules ont été mises en culture. Pour cela, une suspension à $2,5 \, 10^5$ cellules/ml dans du milieu MEM, 10% de sérum de veau foetal (SVF), 0.5% gentamycine a été préparée, et 5 plaques de 96 cupules avec 200 $\mu$l/cupule (soit $5.10^4$ cellules/cupule) ont été ensemencées. Les cellules sont incubées à 37°C en présence de 5% de CO2.

Au jour 0, les cellules sont préincubées avec les peptides pendant 2 heures. Pour cela, les peptides indiqués ci-avant ont été dilués à 50 $\mu$g/ml dans du milieu MEM, 10% SVF, 0,5% gentamycine. Les cupules sont vidées par aspiration, puis 100 $\mu$l /cupule de milieu + peptide (une plaque par peptide) sont ajoutés. A titre de contrôle, une plaque avec 100 $\mu$l/cupule de milieu sans peptide pour le titrage de la suspension virale en absence de peptide est préparée. Le mélange est incubé 2 heures à 37°C en présence de 5% de CO2.

Parallèlement, des dilutions de suspensions virales sont préparées, de 10 en 10 jusqu'à $10^{-4}$, puis des dilutions de 4 en 4 jusqu'à $10^{-8.8}$ dans du milieu MEM, sans SVF, 0,5% gentamycine (50 $\mu$l/cupule et 4 cupule/dilution).

Pour l'utilisation lors de l'infection, les peptides sont dilués dans du milieu MEM, 3% SVF, 0,5% gentamycine de façon à obtenir une concentration finale de peptide à 25$\mu$g/ml (sachant que pour le test on ajoute 150 $\mu$ll de milieu contenant le peptide sur 50 $\mu$l de dilution virale).

L'étape d'infection a été réalisée par vidage des cupules (par aspiration) puis ajout des éléments suivants :

- 150 $\mu$l/cupule de peptide dilué ou de milieu pour la plaque non traitée,
- 50 $\mu$l/cupule des dilutions virales $10^{-5.8}$ à $10^{-8.8}$ à raison de 4 cupules/dilution.

Incuber à 37°C pendant 5 jours en présence de 5% de CO2. 5 Jours après l'infection, les titres en DCP50/ml sont déterminés comme décrit ci-avant.

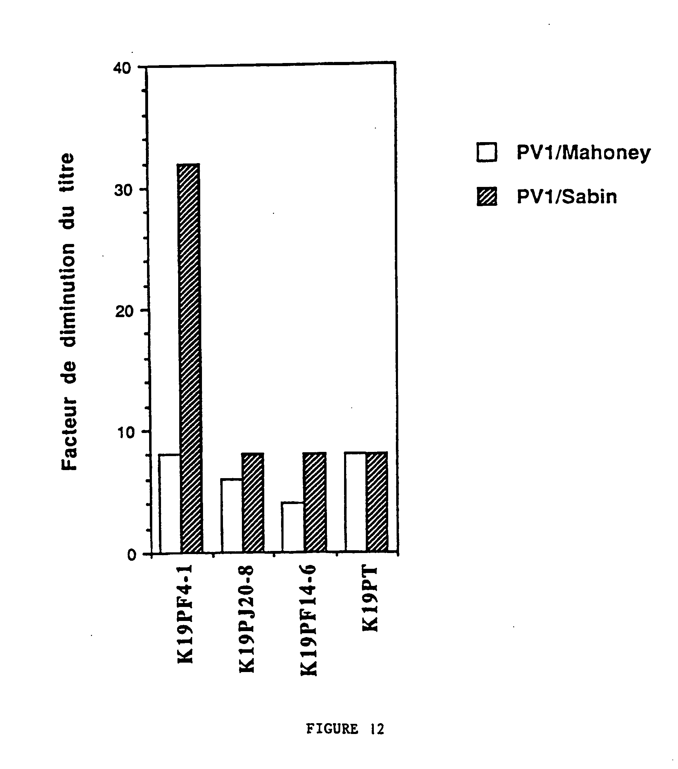

Les résultats obtenus sont présentés dans le Tableau ci-dessous et sur la figure 12. Ces résultats montrent la capacité des polypeptides de l'invention, seuls, à inhiber la réplication de différentes souches de poliovirus.

Tableau

| VIRUS | PEPTIDE | LOG (TITRE/ML) | FACTEUR DE DIMINUTION DU TITRE |
|---|---|---|---|
| PV1/Mahoney | sans | 9,65 | |
| | K19 pF4-1 | 8,75 | 8 |
| | K19 pJ20-8 | 8,9 | 6 |
| | K19 PF14-6 | 9,05 | 4 |
| | K19 PT | 8,75 | 8 |
| PV1/Sabin | sans | 9,5 | |
| | K19 pF4-1 | 8 | 32 |
| | K19 PJ 20-8 | 8,6 | 8 |
| | K19 pF14-6 | 8,6 | 8 |
| | K19 PT | 8,6 | 8 |

En particulier, ces résultats montrent que le peptide K19-pF4.1 possède un effet inhibiteur de la réplication du poliovirus de type 1 très prononcé, d'un facteur supérieur à 30. Cette expérience illustre les multiples applications de la présente invention, à l'inhibition de différents types de virus.

12. Inhibition d la réplication du cytomégalovirus (CMV)

Cet exemple illustre les propriétés antivirales des composés selon l'invention sur le cytomégalovirus.

**Matériel :**

Cellules :

Des fibroblastes diploïdes humains et des astrocytomes humaines primaires (U373 MG) ont été utilisés. Ils sont cultivés dans du milieu Dulbecco additionné de 2mM de glutamine et 10% de sérum de veau foetal.

Peptides :

Le composé utilisé est le dérivé peptidique K19-pF4-1.

Virus :

La souche Ad169 de CMV (ATCC VR538) a été utilisée. La titration du CMV se fait par le comptage des plages formées sous la carboxymethyl-cellulose (0.6%).

Procédé ;

Les cellules ont été traitées par la trypsine, lavées, distribuées dans les puits de plaques de culture à raison de $10^5$ par puits et cultivées pendant 6 à 24 heures. Les polypeptides, en solution stérile, ont été ajoutés aux cultures à une concentration finale de 25 à 50 $\mu$g/ml. Les cellules sont infectées à une multiplicité d'infection de 1 pfu/cellule.
Trois modes opératoires ont été suivis :

a) les polypeptides sont ajoutés 4 heures avant l'infection virale
b) les polypeptides sont ajoutés en même temps que le virus
c) les polypeptides sont ajoutés après l'absorption du virus sur les cellules (2 heures à 37°C).

L'effet antiviral est évalué :

1) visuellement en observant au microscope optique l'aspect morphologique des cellules,
2) en titrant la multiplication virale par comptage des plages formées 5 jours après l'infection, et
3) en analysant par Western-blot l'apparition des protéines très précoces, précoces et tardives de l'infection virale.

**Résultats**

Les résultats obtenus montrent que :

1) Les cellules traitées avec le K19-pF4-1, avant ou en même temps que l'infection virale, ne présentent pas de modifications morphologiques (effets cytopathogènes : ECP). Au contraire, les cellules traitées avec le K19-pF4.1 à la fin de l'adsorption virale présentent des ECP importants à 24 et 48 heures après infection. Les cellules non traitées ou traitées après l'adsorption virale avec le K19-pF4.1 ou de la polylysine libre de 19 résidus (K19) montrent des ECP importants.

2) La réplication du virus est inhibée à 99.5% dans les cellules traitées avec le K19-pF4.1 avant, ainsi que en même temps, que l'infection virale (Voir Tableau ci-dessous).

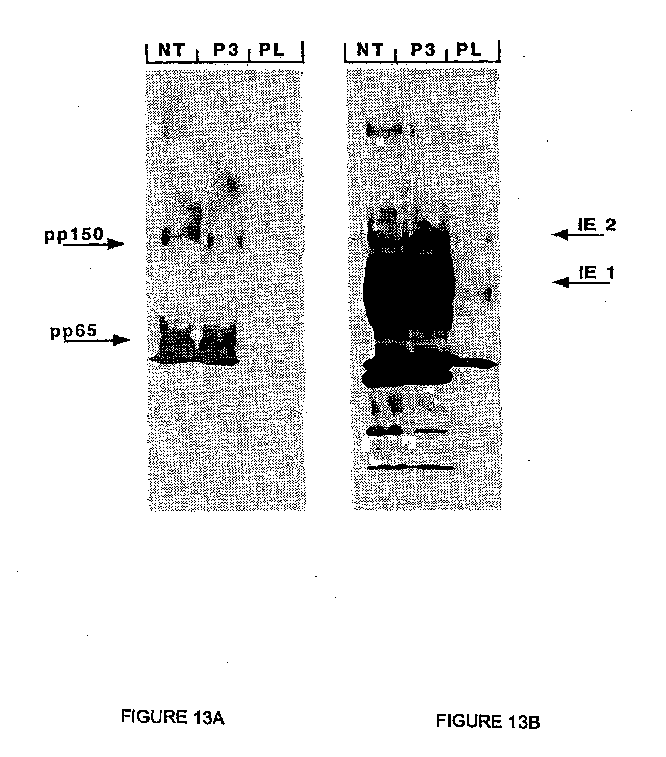

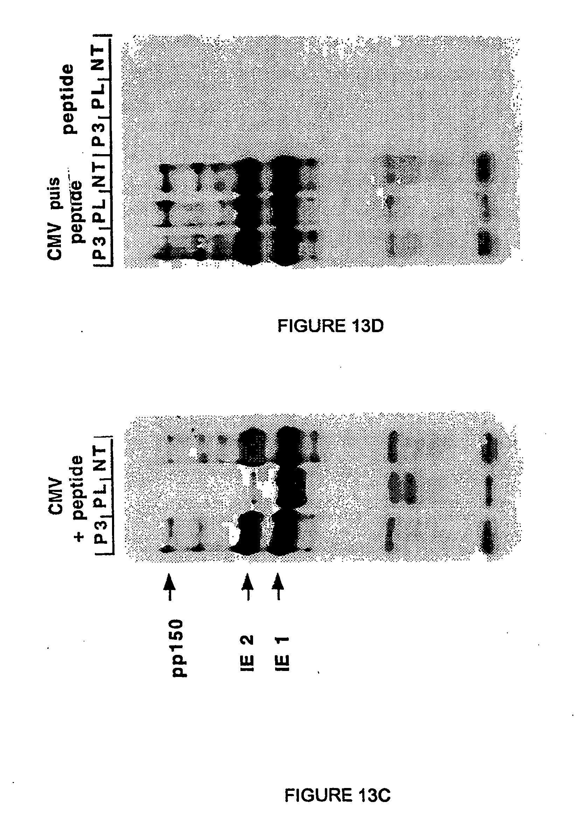

3) Dans les cellules traitées avec K19-F4.1 avant l'infection virale, on ne note pas l'apparition ni des protéines précoces, ni des protéines tardives du CMV (Figures 13a et 13b). Dans les cellules traitées avec K19-pF4.1 en même temps que l'infection virale, on n'observe pas l'apparition des protéines tardives, mais on note l'apparition, cependant diminuée en quantité, des protéines très précoces (Figure 13c). Le K19-pF4.1 n'a pas d'action s'il est ajouté aux cellules après l'adsorption virale (Figure 13d).

En conclusion, les résultats présentés montrent que le polypeptide K19-pF4.1 inhibe significativement la réplication du CMV lorsque les cellules sont traitées avec ce polypeptide avant l'infection virale. Le peptide, lorsqu'il est ajouté aux cellules en même temps que le CMV est aussi efficace, mais peut être, à un moindre degré (apparition d'antigènes précoces).

Tableau

| NOMBRE DE PLAGES FORMEES | | | | | | |
|---|---|---|---|---|---|---|
| Jour 1 | | | | Moyenne | SD | % Reduct |
| no Rx | 50 | 50 | 0 | 33,333 | 228,9 | |
| K19-pF4.1 | 100 | 125 | 75, | 100,0 | 25,0 | |
| Jour 4 | | | | | | |
| no Rx | 18000 | 50750 | 31500 | 33416,7 | 16458,9 | |
| K19-pF4.1 | 0 | 250 | 250 | 166,7 | 144,3 | 99,5 |

Titrages de CMV à 5 jours après infection de cellules prétraitées avec K19-pF4.1 à 25 $\mu$g/ml ou sans traitement (no RX).

13 Inhibition du virus de l'herpès

Cet exemple illustre les propriétés antivirales des composés de l'invention sur le virus de l'herpès simplex.

L'activité antivirale a été déterminée par mesure de l'effet cytopathogène (ECP) induit par un virus HSV-1 TK$^+$ (i.e., exprimant la thymidine kinase) et par un virus HSV-1 TK$^-$ (i.e., n'exprimant pas la thymidine kinase, et donc insensible à l'AZT).

Les peptides et anticorps utilisés sont les suivants : K19, K19-pF4.1, K19-pJ20.8, K19-pF14.6 et F14.6.

L'Acyclovir® a été utilisé comme composé anti HSV-1 TK$^+$ de référence (2 mg, MW 225, Wellcome). L'Acyclovir a été dissout dans 2 ml de milieu DMEM 1X supplémenté par des antibiotiques, puis 50 $\mu$l de NaOH 1N ont été ajoutés. Le volume a été ajusté à 4,44 ml avec du milieu DMEM 1X, AB, et le pH a été ajusté à 7-7,4 au moyen de HCl 1 N. Les solutions neutralisées ont été stérilisées sur des filtres 0,45 $\mu$m, puis conservées sous forme d'aliquots à -20°C. La solution ($10^{-2}$M) a été diluée de 1/10 à $1/10^4$ juste avant emploi (à partir de $10^{-3}$M) et ajoutée à raison de 50 $\mu$l à 1 heure post-infection.

Les stocks viraux (HSV-1 TK$^+$, septième passage sur cellules Vero, et HSV-1 TK-, quatrième passage) ont été dilués au 1/100 dans du milieu de culture préalablement à leur distribution dans les plaques (50$\mu$l / puits).

Le test d'inhibition de l'ECP est un test semi-quantitatif, qui mesure le % de survie des cellules. Le protocole suivant a été utilisé : des cellules Véro ont été trypsinisées (trypsine-Versene, Eurobio), réparties dans des plaques 96 puits à fond plat (Falcon), à raison de 2 x $10^4$ cellules par puits, dans 50 $\mu$l de milieu DMEM (Bioproducts) supplémenté d'antibiotiques, de L-Glutamine et de 5% SVF (Eurobio), puis incubées pendant 24 heures. Les peptides/anticorps, stérilisés sur filtre 0,22 $\mu$M, ont été ajoutés à raison de 50 à 400 $\mu$g/ml (soit de 20 à 2,5 $\mu$g / puits) selon le schéma suivant :

- H-24 : mise en culture des cellules
- HO : Infection
- H-2, H-1, HO, H+1 : Ajout des peptides
- H+48 : Test ECP

Au temps 48 heures post infection, le milieu est retiré et les puits sont lavés 3 fois avec 200 µl de tampon PBS stérile contenant du $Ca^{2+}$ et du $Mg^{2+}$, de manière à éliminer les cellules mortes. Du rouge neutre (RAL, 0,3%) a été ajouté (100 µl/puits) et les cellules ont été incubées pendant 2 heures supplémentaires. Les cellules ont ensuite été lavées 3 fois comme décrit ci-avant pour éliminer le colorant non-incorporé aux cellules. Les cellules ont alors été explosées par ajout de 100 µl de SDS 1% dans l'eau distillée. Après 1 heure à 4°C pour permettre un relargage complet du rouge neutre, les cristaux ont été dissous avec une pipette. Les bulles d'air ont été détruites par courant d'air chaud (sèche-cheveux) et les plaques ont été lues, au moyen d'un lecteur de microplaques, à 570 nm, en utilisant un filtre de référence à 630 nm. Le blanc de densité optique a été réalisé sur l'air.

Les contrôles suivants ont été réalisés :

- cellules témoin : non-infectées et non traitées, incubées avec le rouge neutre ;
- virus témoin : cellules infectées avec différentes dilutions d'un facteur 2 de virus, depuis l'effet ECP 100% (dilution 1/1) à un ECP 12,5% (dilution 1/8) ;
- témoin antiviral : cellules traitées à l'acyclovir et infectées , et
- témoin de toxicité : cellules non-infectées, et traitées avec les différents peptides/anticorps.

L'activité antivirale est déterminée comme suit :

$$AI = 100 \times \frac{(OD \text{ cellule} + Virus + Peptide) - OD \text{ virus témoin } 100\% \text{ ECP}}{OD \text{ cellules témoin} - OD \text{ virus témoin } 100\% \text{ ECP}}$$

Les résultats obtenus sont présentés dans les tableaux qui suivent. Les résultats montrent clairement une inhibition de l'effet ECP induit par un virus HSV-1 TK+ et TK-. L'effet observé est particulièrement prononcé pour le peptide K19-pJ20.8, qui est très actif contre les deux souches virales, notamment lorsqu'il est incubé préalablement ou simultanément à l'infection virale.

L'ensemble des résultats présentés ci-avant démontre donc les propriétés antivirales importantes des anticorps/ peptides de l'invention, seuls ou en combinaison, sur différents types de virus tels que le HIV, le poliovirus, le CMV et HSV-1. Ces résultats illustrent les applications des produits de l'invention pour minimiser, in vitro, ex vivo ou in vivo les effets de l'infection virale.

**Tableaux contrôlés**

| VIRUS TEMOIN | | |
|---|---|---|
| Dilution | HSV1 TK+ | HSV1 TK- |
| 0/0 (cellule contrôle) | 1,292 ± 0,146 | 1,292 ± 0,146 - |
| 1/1 | 0,163 ± 0,060 | 0.297 ± 0,068 |
| 1/2 | 0,206 ± 0,062 | 0,216 ± 0,072 |
| 1/4 | 0,217 ± 0,041 | 0,252 ± 0,058 |
| 1/8 | 0,227 ± 0,039 | 0,289 ± 0,029 |
| TEMOIN ANTIVIRAL (ACYCLOVIR®) | | |
| conc. (M) / HSV | HSV1 TK+ | HSV1 TK- |
| $10^{-3}$/0 | 0,909 ± 0,016 | 0,789 ± 0,160 |
| $10^{-4}$/0 | 1,027 ± 0,042 | 0,834 ± 0,086 |

(suite)

| TEMOIN ANTIVIRAL (ACYCLOVIR®) | | |
|---|---|---|
| 10⁻⁵/0 | 0,975 ± 0,093 | 0,869 ± 0,053 |
| 10⁻⁰/0 | 0,960 ±-0,025 | 0,816 ± 0,001 |
| 10⁻³ / HSV | **0,626 ± 0,050 62%** | 0,289 0,055 0% |
| 10⁻⁴ / HSV | **0,587 ± 0,149 49%** | 0,173 ± 0,001 0% |
| 10⁻⁵ / HSV | **0,223 ± 0,017 7%** | 0,169 ± 0,019 0% |
| 10⁻⁶ / HSV | **0,235 ± 0,032 9%** | 0,239 ± 0,111 0% |

## Tableaux Tests

| HSV1 TK⁺ H-2 | | | |
|---|---|---|---|
| peptide μg/puits | 20 | 10 | 5 |
| K19 | 0 | 0 | 0 |
| K19 pF4-1 | 7 | 2 | 1 |
| K19 PJ20-8 | **78** | **44** | 1 |
| K19 pF14-6 | TOX(*) | TOX | 1 |
| F14-6 | 0 | 0 | 0 |
| (*) Toxique | | | |

| HSV1 Tk⁺ H-1 | | | |
|---|---|---|---|
| peptide - μg/puits | 20 | 10 | 5 |
| K19 | 9 | 6 | 8 |
| K19 pF4-1 | 28 | 30 | 13 |
| K19 PJ20-8 | **89** | **74** | 27 |
| K19 PF14-6 | TOX | TOX | 8 |
| F14-6 | 0 | 0 | 0 |

| HSV1 TK⁺ H0 | | | |
|---|---|---|---|
| peptide - μg/puits | 20 | 10 | 5 |
| K19 | 32 | 14 | 13 |
| K19 pF4-1 | 28 | 30 | 13 |
| K19PJ20-8 | **86** | **71** | 37 |
| K19 pF14-6 | TOX | TOX | **69** |
| F14-6 | 0 | 0 | 0 |

| HSV1 TK⁺ H+1 | | | |
|---|---|---|---|
| peptide - μg/puits | 20 | 10 | 5 |

(suite)

| HSV1 TK⁺ H+1 | | | |
| --- | --- | --- | --- |
| K19 | 0 | 0 | 6 |
| K19 pF4-1 | 10 | 12 | 10 |
| K19PJ20-8 | 0 | 1 | 0 |
| K19 pF 14-6 | TOX | TOX | 37 |
| F14-6 | 0 | 0 | 0 |

| HSV1 TK⁻ H-2 | | | |
| --- | --- | --- | --- |
| peptide - μg/puits | 20 | 10 | 5 |
| K19 | 0 | 26 | 27 |
| K19 pF4-1 | 25 | 5 | 21 |
| K19PJ20-8 | 0 | 46 | 55 |
| K 19 PF 14-6 | TOX | TOX | **100** |
| F14-6 | 0 | 21 | 24 |

| HSV1 TK⁻ H-1 | | | |
| --- | --- | --- | --- |
| peptide μg/puits | 20 | 10 | 5 |
| K19 | 34 | 4 | 0 |
| K19 pF4-1 | 47 | 7 | 25 |
| K19 pJ20-8 | **100** | 49 | 29 |
| K19 PF14-6 | TOX | TOX | **72** |
| F 14-6 | 0 | 0 | 0 |

| HSV1 TK⁻ H0 | | | |
| --- | --- | --- | --- |
| peptide - μg/puits | 20 | 10 | 5 |
| K19 | 40 | 2 | 0 |
| K19 pF4-1 | 42 | 25 | 15 |
| K19PJ20-8 | **86** | **56** | 38 |
| K19 pF14-6 | TOX | **92** | **81** |
| F14-6 | 0 | 0 | 0 |

| HSV1 TK⁻ H+1 - | | | |
| --- | --- | --- | --- |
| peptide - μg/puits | 20 | 10 | 5 |
| K19 | 2 | 0 | 0 |
| K19 pF4-1 | 1 | 13 | 1 |
| K19PJ20-8 | 11 | 3 | 1 |

(suite)

| HSV1 TK⁻ H+1 - | | | |
|---|---|---|---|
| K19 pF14-6 | TOX | TOX | 26 |
| F14-6 | 0 | 0 | 0 |

L'ensemble de ces résultats démontre clairement que les polypeptides de l'invention, comportant une région d'un anticorps, de préférence comportant tout ou partie d'un CDR3, sont capables (i) de pénétrer efficacement dans les cellules (ii) d'y véhiculer des substances, en particulier de taille importante (iii), de jouer le rôle d'adjuvant in vivo en stimulant la réponse immunitaire contre un antigène donné et (iv) d'exercer une activité antivirale. En outre, les polypeptides de l'invention semblent même pouvoir transporter des substances jusqu'au noyau des cellules, ce qui représente un intérêt évident lorsque les substances sont des acides nucléiques ou des molécules agissant sur les acides nucléiques. Par ailleurs, les polypeptides de l'invention semblent utiliser un mécanisme de pénétration cellulaire différent de la plupart des vecteurs utilisés jusqu'à présent. En particulier, les polypeptides de l'invention semblent échapper aux lysosomes, ce qui constitue un avantage supplémentaire dans la mesure où un phénomène de dégradation important se produit généralement dans ces compartiments cellulaires.

**Revendications**

1. Polypeptide capable de pénétrer dans les cellules, **caractérisé en ce qu'**il consiste en une seule chaîne d'acides aminés d'au plus 60 résidus comprenant :

   (a) une région CDR2 de séquence (Val)$_m$-(Ala)$_m$-Tyr-Ile-Ser-Arg-Gly-Gly-Val-Ser-Thr-Tyr-Tyr-Ser-Asp-Thr-Val-Lys-Gly-(Arg)$_m$-(Phe)$_m$-(Thr)$_m$ (SEQ ID NO:4), et une région CDR3 de séquence (Thr)$_m$-(Arg)$_m$-(Gln)$_m$-Lys-Tyr-Asn-Lys-Arg-Ala-(Met-Asp-Tyr-Trp-Gly-Gln-Gly-Thr)$_m$ (SEQ ID NO:1);
   (b) une région CDR2 de séquence (Val)$_m$-(Ala)$_m$-Tyr-Ile-Ser-Arg-Gly-Gly-Gly-Ile-Phe-Tyr-Tyr-Gln-Asp-Ser-Ile-Lys-Gly-(Arg)$_m$-(Phe)$_m$ (SEQ ID NO:5) et une région CDR3 de séquence (Thr)$_m$-(Arg)$_m$-(Gln)$_m$-Lys-Tyr-Gly-Lys-Arg-Gly-(Met-Asp-Tyr-Trp-Gly-Gln-Gly-Thr)$_m$ (SEQ ID NO:2) ;
   (c) une région CDR2 de séquence (Val)$_m$-(Ala)$_m$-Ala-Ile-Ser-Arg-Gly-Gly- . Gly-Tyr-Ser-Tyr-Tyr-Leu-Asp-Ser-Val-Lys-Gly-(Arg)$_m$-(Phe)$_m$-(Thr)$_m$-(Ile)$_m$ (SEQ ID NO:6) et une région CDR3 de séquence (Thr)$_m$-(Arg)$_m$-(Gln)$_m$-Ala-Arg-Ala- Thr-Trp-Asp-Trp-(Phe-Ala-Tyr-Trp-Gly-Gln-Gly-Thr)$_m$ (SEQ ID NO :3);
   (di) une région CDR2 de séquence YI SRGGGIFYYQSIKG et une region CDR3 de séquence EKYGKRGMDY,
   (dii) une région CDR2 de séquence YISRGGVSTYYSDTVKG et une région CDR3 séquence QKYNKRAMDY;
   (diii) une région CDR2 de séquence AISRGGGYSYYLDSVKG et une région CDR3 de séquence TARATWDWFAY
   (e) une partie de la région CDR2 et la région CDR3 telles que définies dans une même option (di) (dii) ou (diii), la partie de la région CDR2 étant obtenue par délétion de 1 à 3 acides aminés terminaux ou par délétion ou substitution conservative de un ou plusieurs résidus d'acides aminés internes de la région CDR2, dans la limite de 15% d'acides aminés modifiés;
   (f) une partie de la région CDR3 et la région CDR2 telles que définies dans une même option (di) (dii) ou (diii), la partie de la région CDR3 étant obtenue par délétion de 1 à 3 acides aminés terminaux ou par délétion ou substitution conservative de un ou plusieurs résidus d'acides aminés internes de la région CDR3, dans la limite de 15% d'acides aminés modifiés; ou
   (g) une partie de la région CDR2 et une partie de la région CDR3 telles que définies dans une même option (di) (dii) ou (diii), les parties des régions CDR2 et CDR3 étant obtenues par déplétion de 1 à 3 acides aminés terminaux ou par délétion ou substitution conservative de un ou plusieurs résidus d'acides aminés internes des régions CDR2 ou CDR3, dans la limite de 15% d'acides aminés modifiés ;

   dans lesquelles m vaut 1 ou 0, et signifie que un seul, plusieurs ou tous les résidus mentionnés dans la parenthèse sont présents ou non.

2. Polypeptide selon la revendication 1, **caractérisé en ce que** les régions CDR2 et CDR3 sont obtenues à partir d'un anticorps polyréactif choisi parmi l'anticorps monoclonal J20.8 produit par l'hybridome déposé à la CNCM sous le numéro CNCM I-1605 et l'anticorps monoclonal F4.1 produit par l'hybridome déposé à la CNCM sous le numéro CNCM I-1606.

**3.** Polypeptide selon la revendication 1 **caractérisé en ce que** la région CDR3 possède une séquence choisie parmi les séquences suivantes : TRQKYNKRA(MDYWGQGT)$_m$. ou TRGARATWDW(FAYWGQGT)$_m$.

**4.** Polypeptide selon l'une quelconque des revendications 1 à 2, **caractérisé en ce que** la région CDR2 possède une séquence choisie parmi les séquences suivantes : VAYISRGGVSTYYSDTVKGRF, VAYISRGGVSTYYSDTVKGRFT, et VAAISRGGGYSYYLDSVKGRFTI.

**5.** Polypeptide selon l'une quelconque des revendications précédentes, **caractérisé en ce qu'**il consiste en la fusion entre la région CDR3 et la région CDR2.

**6.** Polypeptide selon la revendication 5. **caractérisé en ce qu'**il consiste en la séquence Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg Ala-(SEQ ID NO:7) ou la séquence VAYISRGGVSTYYSDTVKGRFTRQKYNKRAVAY.

**7.** Polypeptide en ce qu'il consiste en un polypeptide pénétrant selon l'une quelconque des revendications précédentes et du côté de- terminal, une région polylysine ayant moins de 30 résidus lysine

**8.** Polypeptide, selon la revendication 7, **caractérisé en ce qu'**il est choisi parmi le groupe de polypeptides de séquence suivante :

- (K$_{19}$)-VAYISRGGVSTYYSDTVKGRFTRQKYNKRA (SEQ ID NO :10) ;
- (K$_{19}$)-V-A-Y-I-S-R-G-G-G-I-F-Y-Y-Q-D-S-I-K-G-R-F-T-R-E-K-Y-G-K-R-G-M-D-Y (K19 pFJ20.8); et
- (K$_{19}$)-A-I-S-R-G-G-G-Y-S-Y-Y-L-D-T-V-K-R-T-A-R-A-T-W-D-W-F-A-Y. (K19 pF14.6).

**9.** polypeptide selon l'une quelconque des revendications 1 à 6, **caractérisé en ce qu'**il comprend en outre à une de ses extrémité un résidu cystéine.

**10.** Polypeptide selon la revendication 9, **caractérisé en ce qu'**il consiste en la séquence Cys-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala (SEQ ID NO:9).

**11.** Polypeptide selon l'une quelconque des revendications 1 à 6, **caractérisé en ce qu'**il comprend en outre un groupement biotynyl couplé chimiquement.

**12.** Polypeptide selon la revendication 11, **caractérisé en ce qu'**il consiste en la séquence : Biotinyl-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala.

**13.** Polypeptide selon l'une quelconque des revendications 1 à 12; **caractérisé en ce qu'**il est capable de reconnaître au moins un antigène protéique d'origine cellulaire et/ou virale.

**14.** Polypeptide selon l'une quelconque des revendications 1 à 12; **caractérisé en ce qu'**il a la capacité de réagir *in vitro* avec des macromolécules anioniques comme l'ARN, l'ADN double ou simple brins, ou avec des macromolécules cationiques comme les histones.

**15.** Polypeptide selon l'une quelconque des revendications 1 à 12, ayant la capacité de réagir *in vitro* avec l'héparine et l'héparine sulfate.

**16.** Utilisation d'un polypeptide selon l'une quelconque des revendications 1 à 15, pour la préparation d'une composition destinée au transfert de substances dans les cellules.

**17.** Polypeptide selon l'une quelconque des revendications 1 à 15 auquel est couplée une substance, pour le transfert de ladite substance dans une cellule.

**18.** Polypeptide selon la revendication 17, **caractérisé en ce que** le couplage est un couplage covalent.

**19.** Polypeptide selon la revendication 18, **caractérisé en ce que** le couplage est réalisé par une liaison covalente de type maléimide, succinimide, peptidique, disulfure, amine, acide, biotine-streptavidine ou p-benzoquinone.

**20.** Polypeptide selon la revendication 18, **caractérisé en ce que** ladite substance est un acide nucléique.

**21.** Polypeptide selon la revendication 18, **caractérisé en ce que** ladite substance est une protéine.

**22.** Polypeptide selon la revendication 18, **caractérisé en ce que** ladite substance est un médicament.

**23.** Polypeptide selon la revendication 18, **caractérisé en ce que** ladite substance est un antigène.

**24.** Cellule eucaryote contenant un polypeptide selon l'une quelconque des revendications 1 à 15 et 17 à 23.

**25.** Procédé pour transférer une substance dans une cellule *in vitro* comprenant

- le couplage entre ladite substance et un polypeptide selon l'une des revendications 1 à 15 et,
- l'incubation de la cellule avec le produit dudit couplage.

**26.** Procédé selon la revendication 25, **caractérisé en ce que** l'incubation de la cellule avec le produit du couplage est effectuée en présence de glycérol.

**27.** Composition pharmaceutique comprenant, en association avec un véhicule physiologiquement acceptable, un polypeptide selon la revendication 17 dans lequel la substance est un principe actif de médicament.

**28.** Vaccin comprenant, en association avec un véhicule physiologiquement acceptable, un polypeptide selon la revendication 17 dans lequel la substance est un antigène.

**29.** Polypeptide selon l'une des revendications 1 à 15, pour l'utilisation comme agent antiviral.

**30.** Composition comprenant des cellules incubées ex vivo en présence d'un ou plusieurs polypeptides selon l'une des revendications 1 à 15.

**31.** Composition selon la revendication 30, **caractérisée en ce que** les cellules sont des cellules humaines.


**Claims**

**1.** A polypeptide capable of penetrating into cells, **characterized in that** it consists of a single chain of amino acids of at most 60 residues comprising:

(a) a CDR2 region with sequence $(Val)_m$-$(Ala)_m$-Tyr-Ile-Ser-Arg-Gly-Gly-Val-Ser-Thr-Tyr-Tyr-Ser-Asp-Thr-Val-Lys-Gly-$(Arg)_m$-$(Phe)_m$-$(Thr)_m$ (SEQ ID NO: 4), and a CDR3 region with sequence $(Thr)_m$-$(Arg)_m$-$(Gln)_m$-Lys-Tyr- Asn-Lys-Arg-Ala-$(Met-Asp-Tyr-Trp-Gly-Gln-Gly-Thr)_m$ (SEQ ID NO: 1);
(b) a CDR2 region with sequence $(Val)_m$-$(Ala)_m$-Tyr-Ile-Ser-Arg-Gly-Gly-Gly-Ile-Phe-Tyr-Tyr-Gln-Asp-Ser-Ile-Lys-Gly-$(Arg)_m$-$(Phe)_m$ (SEQ ID NO: 5) and a CDR3 region with sequence $(Thr)_m$-$(Arg)_m$-$(Gln)_m$-Lys-Tyr-Gly-Lys-Arg-Gly-$(Met-Asp-Tyr-Trp-Gly-Gln-Gly-Thr)_m$ (SEQ ID NO: 2);
(c) a CDR2 region with sequence $(Val)_m$-$(Ala)_m$-Ala-Ile-Ser-Arg-Gly-Gly-Gly-Tyr-Ser-Tyr-Tyr-Leu-Asp-Ser-Val-Lys-Gly-$(Arg)_m$-$(Phe)_m$-$(Thr)_m$-$(Ile)_m$ (SEQ ID NO: 6) and a CDR3 region with sequence $(Thr)_m$-$(Arg)_m$-$(Gln)_m$-Ala-Arg-Ala-Thr-Trp-Asp-Trp-$(Phe-Ala-Tyr-Trp-Gly-Gln-Gly-Thr)_m$ (SEQ ID NO: 3);
(di) a CDR2 region with sequence YISRGGGIFYYQDSIKG and a CDR3 region with sequence EKYGKRGMDY;
(dii) a CDR2 region with sequence YISRGGVSTYYSDTVKG and a CDR3 region with sequence QKYNKRAMDY;
(diii) a CDR2 region with sequence AISRGGGYSYYLDSVKG and a CDR3 region with sequence TARATWDWFAY;
(e) a portion of the CDR2 region and the CDR3 region as defined in a same option (di), d(ii) or d(iii), the portion of the CDR2 region being obtained by deletion of 1 to 3 terminal amino acids or by deletion or conservative substitution of one or more internal amino acid residues of the CDR2 region up to a limit of 15% of modified amino acids;
(f) a portion of the CDR3 region and the CDR2 region as defined in a same option (di), d(ii) or d(iii), the portion of the CDR3 region being obtained by deletion of 1 to 3 terminal amino acids or by deletion or conservative substitution of one or more internal amino acid residues of the CDR3 region up to a limit of 15% of modified amino acids; or

27

(g) a portion of the CDR2 region and a portion of the CDR3 region as defined in a same option (di), d(ii) or d (iii), the portions of the CDR2 and CDR3 regions being obtained by deletion of 1 to 3 terminal amino acids or by deletion or conservative substitution of one or more internal amino acid residues of the CDR2 or CDR3 regions up to a limit of 15% ofmodified amino acids;

in which m is 1 or 0, and means that just one, several or all of the residues mentioned in parentheses are present or absent.

**2.** A polypeptide according to claim 1, **characterized in that** the CDR2 and CDR3 regions are obtained from a polyreactive antibody selected from the monoclonal antibody J20.8 produced by the hybridoma deposited at the CNCM with accession number CNCM I-1605 and the monoclonal antibody F4.1 produced by the hybridoma deposited at the CNCM with accession number CNCM I-1606.

**3.** A polypeptide according to claim 1, **characterized in that** the CDR3 region has a sequence selected from the following sequences:

TRQKYNKRA(MDYWGQGT)$_m$ and TRGARATWDW(FAYWGQGT)$_m$.

**4.** A polypeptide according to claim 1 or claim 2, **characterized in that** the CDR2 region has a sequence selected from the following sequences:

VAYISRGGVSTYYSDTVKGRF, VAYISRGGVSTYYSDTVKGRFT and VAAISRGGGYSYYLDSVKGRFTI.

**5.** A polypeptide according to any one of the preceding claims, **characterized in that** it consists of a fusion between the CDR3 region and the CDR2 region.

**6.** A polypeptide according to claim 5, **characterized in that** it consists of the sequence Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala (SEQ ID NO: 7) or the sequence VAYISRGGVSTYYSDTVKGRFTRQKYNKRAVAY.

**7.** A polypeptide, **characterized in that** it consists of a penetrating polypeptide according to any one of the preceding claims and, on the N-terminal side, a polylysine region having less than 30 lysine residues.

**8.** A polypeptide according to claim 7, **characterized in that** it is selected from the group of polypeptides with the following sequence:

• -(K$_{19}$)-VAYISRGGVSTYYSDTVKGRFTRQKYNKRA (SEQ ID NO: 10);
• (K)$_{19}$-V-A-Y-I-S-R-G-G-G-I-F-Y-Y-Q-D-S-I-K-G-R-F-T-R-E-K-Y-G-K-R-G-M-D-Y (K19pFJ20.8); and
• (K)$_{19}$-A-I-S-R-G-G-G-Y-S-Y-Y-L-D-T-V-K-R-T-A-R-A-T-W-D-W-F-A-Y (K19pF14.6).

**9.** A polypeptide according to any one of claims 1 to 6, **characterized in that** it further comprises a cystein residue at one of its ends.

**10.** A polypeptide according to claim 9, **characterized in that** it consists of the sequence Cys-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala-(SEQ ID NO: 9).

**11.** A polypeptide according to any one of claims 1 to 6, **characterized in that** it further comprises a chemically coupled biotynyl group.

**12.** A polypeptide sequence according to claim 11, **characterized in that** it consists of the sequence: biotinyl-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-  Lys-Tyr-Asn-Lys-Arg-Ala.

**13.** A polypeptide according to any one of claims I to 12, **characterized in that** it is capable of recognizing at least one protein antigen of cellular and/or viral origin.

**14.** A polypeptide according to any one of claims 1 to 12, **characterized in that** it has the capacity to react *in vitro* with anionic macromolecules such as RNA, double or single strand DNA or with cationic macromolecules such as histones.

**15.** A polypeptide according to any one of claims I to 12, having the capacity of reacting *in vitro* with heparin and heparin sulphate.

**16.** Use of a polypeptide according to any one of claims 1 to 15, for the preparation of a composition intended to transfer substances into cells.

**17.** A polypeptide according to any one of claims 1 to 15 to which a substance is coupled, for transferring said substance into a cell.

**18.** A polypeptide according to claim 17, **characterized in that** the coupling is covalent coupling.

**19.** A polypeptide according to claim 18, **characterized in that** coupling is carried out by a covalent maleimide, succinimide, peptide, disulphide, amine, acid, biotin-streptavidin or p-benzoquinone type bond.

**20.** A polypeptide according to claim 18, **characterized in that** said substance is a nucleic acid.

**21.** A polypeptide according to claim 18, **characterized in that** said substance is a protein.

**22.** A polypeptide according to claim 18, **characterized in that** said substance is a drug.

**23.** A polypeptide according to claim 18, **characterized in that** said substance is an antigen.

**24.** A eukaryotic cell containing a polypeptide according to any one of claims 1 to 15 and 17 to 23.

**25.** A method for transferring a substance into a cell *in vitro,* comprising:

• coupling said substance and a polypeptide according to one of claims 1 to 15; and
• incubating the cell with the product of said coupling.

**26.** A method according to claim 25, **characterized in that** the cell is incubated with the coupling product in the presence of glycerol.

**27.** A pharmaceutical composition comprising a polypeptide according to claim 17 in which the substance is an active principle of a drug, in association with a physiologically acceptable vehicle.

**28.** A vaccine comprising a polypeptide according to claim 17 in which the substance is an antigen, in association with a physiologically acceptable vehicle.

**29.** A polypeptide according to one of claims I to 15, for use as an antiviral agent.

**30.** A composition comprising cells incubated *ex vivo* in the presence of one or more polypeptides according to one of claims I to 15.

**31.** A composition according to claim 30, **characterized in that** the cells are human cells.

**Patentansprüche**

**1.** Polypeptid, das Fähig ist in Zellen einzudringen, **dadurch gekennzeichnet, dass** es aus einer einzigen Aminosäurenkette aus maximal 60 Aminosäureresten besteht, umfassend:

(a) eine CDR2 Region der Sequenz $(Val)_m$-$(Ala)_m$-Tyr-Ile-Ser-Arg-Gly-Gly-Val-Ser-Thr-Tyr-Tyr-Ser-Asp-Thr-Val-Lys-Gly-$(Arg)_m$-$(Phe)_m$-$(Thr)_m$ (SEQ ID NO :4), und eine CDR3 Region der Sequenz $(Thr)_m$-$(Arg)_m$-$(Gln)_m$-Lys-Tyr-Asn-Lys-Arg-Ala-(Met-Asp-Tyr-Trp-Gly-Gln-Gly-Thr)$_m$ (SEQ ID NO :1) ;
(b) eine CDR2 Region der Sequenz $(Val)_m$- $(Ala)_m$-Tyr-Ile-Ser-Arg-Gly-Gly-Gly-Ile-Phe-Tyr-Tyr-Gln-Asp-Ser-Ile-Lys-Gly-$(Arg)_m$-$(phe)_m$ (SEQ ID NO :5) und eine CDR3 Region der Sequenz $(Thr)_m$-$(Arg)_m$-$(Gln)_m$-Lys-Tyr-Gly-Lys-Arg-Gly-(Met-Asp-Tyr-Trp-Gly-Gln-Gly-Thr)$_m$ (SEQ ID NO :2) ;
(c) eine CDR2 Region der Sequenz $(Val)_m$-$(Ala)_m$-Ala-Ile-Ser-Arg-Gly-Gly-Gly-Tyr-Ser-Tyr-Tyr-Leu-Asp-Ser-Val-Lys-Gly-(Arg)$_m$-(Phe)$_m$-(Thr)$_m$-(Ile)$_m$ (SEQ ID NO :6) und eine CDR3 Region der Sequenz (Thr)$_m$-(Arg)$_m$-(Gln)$_m$-Ala-Arg-Ala-Thr-Trp-Asp-Trp-(Phe-Ala-Tyr-Trp-Gly-Gln-Gly-Thr)$_m$ (SEQ ID NO :3) ;

(di) eine CDR2 Region der Sequenz YISRGGGIFYYQDSIKG und eine CDR3 Region der Sequenz EKYGKRGMDY ;

(dii) eine CDR2 Region der Sequenz YISRGGVSTYYSDTVKG und eine CDR3 Region der Sequenz QKYNKRAMDY ;

(diii) eine CDR2 Region der Sequenz AISRGGGYSYYLDSVKG und eine CDR 3 Region der Sequenz TARATWDWFAY ;

(e) ein Teil der CDR2 Region und die CDR3 Region wie unter einer bestimmten Option (di), (dii) oder (diii) definiert, wobei der Teil der CDR2 Region durch Deletion von 1 bis 3 terminale Aminosäuren oder durch Deletion oder konservative Substitution von einem oder mehreren internen Aminosäureresten der CDR2 Region erhalten wird, wobei die Grenze bei 15% geänderten Aminosäuren liegt ;

(t) ein Teil der CDR3 Region und die CDR2 Region wie unter einer bestimmten Option (di), (dii) oder (diii) definiert, wobei der Teil der CDR3 Region durch Deletion von 1 bis 3 terminalen Aminosäuren oder durch Deletion oder konservative Substitution von einem oder mehreren internen Aminosäureresten der CDR3 Region erhalten wird, wobei die Grenze bei 15% geänderten Aminosäuren liegt;

(g) ein Teil der CDR2 Region und ein Teil der CDR3 Region wie unter einer bestimmten Option (di), (dii) oder (diii) definiert, wobei die Teile der CDR2 und der CDR3 Regionen durch Deletion von 1 bis 3 terminalen Aminosäuren oder durch Deletion oder konservative Substitution von einem oder mehreren internen Aminosäureresten der CDR2 oder der CDR3 Regionen erhalten werden, wobei die Grenze bei 15% geänderten Aminosäuren liegt ;

wobei m 1 oder 0 ist und bedeutet, dass eine einzige, mehrere oder alle Reste innerhalb der Klammern vorhanden sind oder nicht.

2. Polypeptid gemäss Anspruch 1, **dadurch gekennzeichnet, dass** die CDR2 und CDR3 Regionen von einem polyreaktiven Antikörper erhalten werden, der ausgewählt wird aus dem vom unter der Nummer CNCM I-1605 beim CNCM hinterlegten Hybridom produzierten monoklonalen Antikörper J20.8 und dem vom unter der Nummer CNCM I-1606 beim CNCM hinterlegten Hybridom produzierten, monoklonalen Antikörper F4.1.

3. Polypeptid gemäss Anspruch 1, **dadurch gekennzeichnet, dass** die CDR3 Region eine aus den folgenden Sequenzen ausgewählte Sequenz besitzt TRQKYNKRA (MDYWGQGT)$_m$ oder TRGARATWDW(FAYWGQGT)$_m$.

4. Polypeptid gemäss einem beliebigen der Ansprüche 1 bis 2, **dadurch gekennzeichnet, dass** die CDR2 Region eine aus den folgenden Sequenzen ausgewählte Sequenz besitzt : VAYISRGGVSTYYSDTVKGRF, VAYISRGGVSTYYSDTVKGRFT, und VAAISRGGGYSYYLDSVKGRFTI.

5. Polypeptid gemäss einem beliebigen der vorangehenden Ansprüche, **dadurch gekennzeichnet, dass** es aus der Fusion der CDR3 Region und der CDR2 Region besteht.

6. Polypeptid gemäss Anspruch 5, **dadurch gekennzeichnet, dass** es aus der Sequenz Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala (SEQ ID NO :7) oder der Sequenz VAYISRGGVSTYYSDTVKGRFTRQKYNKRAVAY besteht.

7. Polypeptid, **dadurch gekennzeichnet, dass** es aus einem eindringenden Polypeptid gemäss einem beliebigen der vorangehenden Ansprüche und, an dessen N-terminaler Seite, einer Polylysineregion mit mindestens 30 Lysinresten besteht.

8. Polypeptid gemäss Anspruch 7, **dadurch gekennzeichnet, dass** es aus der Gruppe von Polypeptiden der folgenden Sequenz ausgewählt ist :

- (K$_{19}$)-VAYISRGGVSTYYSDTVKGRFTRQKYNKRA (SEQ ID NO : 10) ;
- (K$_{19}$)-V-A-Y-I-S-R-G-G-G-I-F-Y-Y-Q-D-S-I-K-G-R-F-T-R-E-K-Y-G-K-R-G-M-D-Y (k19 pFJ20.8); und
- (K$_{19}$)-A-I-S-R-G-G-G-Y-S-Y-Y-L-D-T-V-K-R-T-A-R-A-T-W-D-W-F-A-Y (K19 pF14.6).

9. Polypeptid gemäss einem beliebigen der Ansprüche 1 bis 6, **dadurch gekennzeichnet, dass** es ausserdem an einem seiner Enden einen Cysteinrest umfasst.

10. Polypeptid gemäss Anspruch 9, **dadurch gekennzeichnet, dass** es aus der Sequenz Cys-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala   (SEQ   ID   NO :9) besteht.

11. Polypeptid gemäss einem beliebigen der Ansprüche 1 bis 6, **dadurch gekennzeichnet, dass** es ausserdem eine chemisch gebundene biotinyl-Gruppe umfasst.

12. Polypeptid gemäss Anspruch 11, **dadurch gekennzeichnet, dass** es aus der Sequenz :

   Biotinyl-Val-Ala-Tyr-Ile-Ser-Arg-Gly-Gly-Val-Ser-Thr-Tyr-Tyr-Ser-Asp-Thr-Val-Lys-Gly-Arg-Phe-Thr-Arg-Gln-Lys-Tyr-Asn-Lys-Arg-Ala besteht.

13. Polypeptid gemäss einem beliebigen der Ansprüche 1 bis 12, **dadurch gekennzeichnet, dass** es fähig ist mindestens ein Proteinantigen zellulärer und/oder viraler Herkunft zu erkennen.

14. Polypeptid gemäss einem beliebigen der Ansprüche 1 bis 12, **dadurch gekennzeichnet, dass** es die Fähigkeit hat *in vitro* mit anionischen Makromolekülen wie RNS, doppeloder einsträniger DNS, oder mit kationischen Makromoleküle wie Histone zu reagieren.

15. Polypeptid gemäss einem beliebigen der Ansprüche 1 bis 12, welches die Fähigkeit hat *in vitro* mit Heparin oder Heparinsulfat zu reagieren.

16. Verwendung eines Polypeptids gemäss einem beliebigen der Ansprüche 1 bis 15 zur Herstellung einer zum Transfer von Substanzen in Zellen bestimmten Zusammensetzung.

17. Polypeptid gemäss einem beliebigen der Ansprüche 1 bis 15, an das eine Substanz gebinden ist, zum Transfer der besagten Substanz in eine Zelle.

18. Polypeptid gemäss Anspruch 17, **dadurch gekennzeichnet, dass** die Bindung eine kovalente Bindung ist.

19. Polypeptid gemäss Anspruch 18, **dadurch gekennzeichnet, dass** die Bindung durch eine kovalente Bindung des Maleimid-, Succinimid-, Peptid-, Disulfd-, Amin-, Säure-, Biotin-Streptavidine- oder p-Benzochinontyps realisiert ist.

20. Polypeptid gemäss Anspruch 18, **dadurch gekennzeichnet, dass** die besagte Substanz eine Nukleinsäure ist.

21. Polypeptid gemäss Anspruch 18, **dadurch gekennzeichnet, dass** die besagte Substanz ein Protein ist.

22. Polypeptid gemäss Anspruch 18, **dadurch gekennzeichnet, dass** die besagte Substanz ein Medikament ist.

23. Polypeptid gemäss Anspruch 18, **dadurch gekennzeichnet, dass** die besagte Substanz ein Antigen ist.

24. Eukariotische Zelle, die ein Polypeptid gemäss einem beliebigen der Ansprüche 1 bis 15 5 und 17 bis 23 enthält.

25. Verfahren zum Transfer einer Substanz in eine Zelle *in vitro* umfassend

   - das miteinander Verbinden der besagten Substanz und eines Polypeptids gemäss einem der Ansprüche 1 bis 15 und,
   - die Inkubation der Zelle mit dem Erzeugnis des besagten Verbindens.

26. Verfahren gemäss Anspruch 25, dadurch gekemizeichnet, dass die Inkubation der Zelle mit dem Produkt des Verbindens in der Gegenwart von Glycerol durchgeführt wird.

27. Pharmazeutische Zusammensetzung umfassend, zusammen mit einem physiologisch akzeptierbaren Träger, ein Polypeptid gemäss Anspruch 17, wobei die Substanz ein aktives Prinzip eines Medikamentes ist.

28. Impfstoff umfassend, zusammen mit einem physiologisch akzeptierbaren Träger, ein Polypeptid gemäss Anspruch 17, wobei die Substanz ein Antigen ist.

29. Polypeptid gemäss einem der Ansprüche 1 bis 15, zur Verwendung als antivirales Mittel.

30. Zusammensetzung umfassend Zellen, die *ex vivo* in Gegenwart eines oder mehrerer Polypeptide gemäss einem der Ansprüche 1 bis 15 inkubiert sind.

31. Zusammensetzung gemäss Anspruch 30, **dadurch gekennzeichnet, dass** die Zellen menschliche Zellen sind.

FIGURE 1

```
                                        CDR1                    CDR2
                                                          50  52A    60
        10        20        30          40
         GGSLKLSCAASGFTFS SYAMS WVRQTPAKRLEWVA YISRGGGIFYYQDSIKG RFTI
J20.8    
F4.1     ET------------------------------------------VST--S-TV-----
F14.6    ALVKP--------------N-G--------E----------A-------YS--L--V-----
H9.3     ELVR--A-V-V-CTT---NIK DDYIH----R-EQG---IG R-DPAN-KTK-APKFQD KA--
A2.1     GLVKP-A-V-V--NV--YS-T G-F-N----SHG-S----G R-NPLN-DTF-NQKF--KA-L

                                     CDR3
        70        80   ABC      90
         ARDNAKNTLYLQMSSLRSEDTAMYYCTR EKY..GKRG.MDY WGQGTSVTVSS
J20.8    
F4.1     S---------S------------------A- Q--N..--A.------------
F14.6    S----R--------------E-------A- TAR..ATWDWFA- -----L----A
H9.3     TA-TSS--A---L---T-----V---S- SNYYGNSPSWFA- ---------A
A2.1     TV-KSS--AHMELR--K--NS-V--CA- GL...T-WYF-V -A--T--L-A
```

FIGURE 2

34

```
        CDR2                              CDR3

YISRGGGIFYYQDSIKG          EKYGKRG      MDY (J20.8)
------VST--S-TV--          QKYNKRA      MDY (F4.1)
A------YS--L--V--          TARATWDW     FAY (F14.6)
```

FIGURE 3

## Réponse anti-streptavidine

FIGURE 4A

## Réponse anti-peroxydase

FIGURE 4B

FIGURE 5

FIGURE 6

38

FIGURE 7

présence de glycérol

absence de glycérol

□ pLK$_{19}$

□ CW-K$_{19}$

■ K$_{19}$-P3

▨ PEI

FIGURE 8A

FIGURE 8B

absence de glycérol

FIGURE 9A

□ pLK$_{19}$

□ CW-K$_{19}$

■ K$_{19}$-P3

◫ PEI

présence de glycérol

FIGURE 9B

ACTIVITE ANTIVIRALE DES
ANTICORPS ANTI-ADN

FIGURE 10

FIGURE I IA

## TITRES INFECTIEUX AU JOUR 13
### Exp III

FIGURE 11B

## Titres infectieux au jour 13
## Exp V

FIGURE IIC

FIGURE 12

FIGURE 13A                    FIGURE 13B

FIGURE 13D

FIGURE 13C

## RÉFÉRENCES CITÉES DANS LA DESCRIPTION

**Documents brevets cités dans la description**

- WO 9702840 A
- US 5635383 A
- FR 9508316
- FR 7537392
- US 4925921 A

**Littérature non-brevet citée dans la description**

- The Structure of A Typical Antibody Molecule. **C. JANEWAY ; P. TRAVERS.** immunobiology. Academic Press, 1996
- **T.T. WU ; E. KABAT.** *J. Exp. Med.,* 1970, vol. 132, 211-250
- **ALARCON-SEGOVIA et al.** *Nature,* 1978, 271
- **VLAHAKOS et al.** *J. Am. Soc. Nephrol.,* 1992, vol. 2, 1345
- **EYAL RAZ et al.** *Eur. J. Immunol.,* 1993, vol. 23, 383
- **OKUDAIRA et al.** *Arthritis and Rheumatism,* 1987, vol. 30, 669
- **SIBILLE et al.** *Eur. J. Immunol.,* 1997, vol. 27, 1221-1228
- **GREG. T._ HERMANSON.** Bioconjugate Techniques. Academic Press, 1996
- **S.L. MORRISO ; V.T. OI.** *Advances in Immunology,* 1989, vol. 44, 65-92
- **J.G.R. HURRELL.** Monoclonal Hybridoma Antibiotics : techniques and Applications. CRC Press, 1982
- **EY et al.** *Immunochemistry,* 1978, vol. 15, 429
- **GUILBERT et al.** *J. Immunol.,* 1982, vol. 128, 2779
- **SCHWARTZ et al.** *Biol. Cell,* 1991, vol. 73, 7
- **DEVEREUX J.** *The GCG Séquence Analysis Software Package,* 1989
- **WADHWA et al.** *Bioconjugate Chen.,* 1997, vol. 8, 81-88
- Enteroviruses. **MELNICK et al.** Diagnostic procedure for viral, rickettsial and chlamydial infections. American Public Health Association, 1979, 471-534